# EUROPEAN PATENT APPLICATION

(11) **EP 3 034 496 A1**
(43) Date of publication of application: **22.06.2016**
(21) Application number: 14198980.6
(22) Date of filing: 18.12.2014
(51) Int. Cl.: C07D 209/48, A61K 31/4035, A61P 35/00

(54) **NOVEL SOLVATES OF N-[2-[(1S)-1-(3-ETHOXY-4-METHOXYPHENYL)-2-(METHYLSULFONYL)ETHYL]-2,3-DIHYDRO-1,3-DIOXO-1H-ISOINDOL-4-YL]ACETAMIDE**

(71) Applicant: LEK Pharmaceuticals d.d., 1526 Ljubljana (SI)
(72) Inventor: The designation of the inventor has not yet been filed
(74) Representative: Kluschanzoff, Harald

(57) **Abstract**

The present invention relates to novel crystalline solvates of *N*-[2-[(1*S*)-1-(3-ethoxy-4-methoxyphenyl)-2-(methylsulfonyl)ethyl]-2,3-dihydro-1,3-dioxo-1 H-isoindol-4-yl]acetamide and methods for their preparation. Furthermore, the invention concerns pharmaceutical compositions comprising an effective amount of one or more of the novel crystalline solvates and the use thereof as medicaments.

## Description

### FIELD OF THE INVENTION

The present invention relates to novel crystalline solvates of *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide and methods for their preparation. Furthermore, the invention concerns pharmaceutical compositions comprising an effective amount of one or more of the novel crystalline solvates and the use of said pharmaceutical compositions as medicaments.

### BACKGROUND OF THE INVENTION

*N*-[2-[(1*S*)-1-(3-ethoxy-4-methoxyphenyl)-2-(methylsulfonyl)ethyl]-2,3-dihydro-1,3-dioxo-1*H-*isoindol-4-yl]acetamide (international non-proprietary name: apremilast) represented by the chemical structure (I) 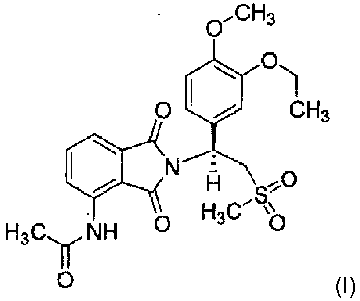 is an inhibitor of phosphodiesterase IV (PDE4) and currently indicated for the treatment of active psoriatic arthritis and moderate to severe plaque psoriasis.

WO 2003/080049 A1 discloses the compound *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide and methods for its preparation. Example 2 provides on page 29, lines 1 to 21 a specific process for the preparation of *N*-[2-[(1*S*)-1-(3-ethoxy-4-methoxyphenyl)-2-(methylsulfonyl)ethyl]-2,3-dihydro-1,3-dioxo-1*H-*isoindol-4-yl]acetamide comprising a final recrystallization step from a binary solvent containing ethanol and acetone.

WO 2009/120167 A1 discloses several crystalline forms of *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide and methods for their preparation. The non-solvated anhydrous forms A, B and F are disclosed, as well as several solvates with organic solvents selected from toluene (form C), methylene chloride (form D), acetonitrile (form E), ethyl acetate (form G) and tetrahydrofuran (form H).

However, the so far disclosed solvates of *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide have certain drawbacks. For example most of these solvates contain solvents, which should be limited in pharmaceutical products due to their toxic potential. In addition, majorities of the known solvates contain, and are therefore crystallized from, water-immiscible organic solvents. As organic solvents are expensive and harmful to the environment they should be reduced to the extent possible e.g. by partly replacing them with water, which favors the use of water-miscible organic solvents for the final crystallization step. Moreover, according to WO 2009/120167 A1 the therein disclosed solvates consist of very small particles, which makes pharmaceutical development of a drug product challenging due to poor powder characteristics.

Hence, there is a need for novel crystalline solvates of *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide overcoming the issues associated with the so far known solvates. In particular, there is a need for novel solvates of *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide comprising less toxic and water-miscible organic solvents, which can be prepared in an economical and green manner. In addition, there is a need for solvates of *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide having improved powder characteristics and for pharmaceutical compositions comprising the same.

### SUMMARY OF THE INVENTION

The present invention relates to novel crystalline solvates of *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide and to processes for their preparation. The novel solvates contain less toxic organic solvents with high water solubility. Consequently, the solvates of the present invention can be used in pharmaceutical drug products and produced in a green and economic manner. Furthermore, the novel solvates of the present invention show improved powder characteristics and are therefore preferably used in pharmaceutical production processes.

The present invention in particular provides various aspects, advantageous features and preferred embodiments as summarized in the following items, which respectively alone or in combination particularly contribute to solving the object of the invention and eventually provide additional advantages:
(1) A crystalline solvate of *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide and an organic solvent, wherein the organic solvent is selected from an ICH class 3 solvent which has a water solubility of at least 20% w/w when measured at a temperature in the range of from 15 to 25 °C.

The term "crystalline solvate" as used herein refers to a solid where an organic solvent is coordinated in or accommodated by the crystal structure.

The term "ICH class 3 solvent" as used herein refers to solvents listed in table 3 of ICH HARMONIZED TRIPARTITE GUIDELINE, impurities: guideline for residual solvents Q3C(R5), current step 4 version, February 4th, 2011. These solvents are classified as solvents with low toxic potential and it is considered that amounts of these solvents of 50 mg per day or less would be acceptable without justification.
(2) The crystalline solvate according to item 1, wherein the organic solvent is selected from the group consisting of ethanol, n-propanol, acetone and methyl ethyl ketone.
(3) The crystalline solvate according to item 1, wherein the organic solvent is ethanol and which is characterized by having a powder X-ray diffractogram comprising reflections at 2-Theta angles of (7.4 ± 0.2)°, (11.2 ± 0.2)°, (15.2 ± 0.2)°, (16.3 ± 0.2)°, (26.3 ± 0.2)°, when measured at a temperature in the range of from 15 to 25 °C with Cu-Kalpha_{1,2} radiation having a wavelength of 0.15419 nm.
(4) The crystalline solvate according to item 3, having a Fourier transform infrared spectrum comprising peaks at wavenumbers of (1762 ± 2) cm⁻¹, (1696 ± 2) cm⁻¹, (1618 ± 2) cm⁻¹, (1519 ± 2) cm⁻¹, (1135 ± 2) cm⁻¹, when measured at a temperature in the range of from 15 to 25 °C with a ZnSe ATR cell.
(5) The crystalline solvate according to item 3 or 4, which is characterized by having a crystal structure comprising 0.5 molecules ethanol per molecule *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide, as determined by an X-ray single crystal structure analysis at 293 K.
(6) The crystalline solvate according to any one of items 3 to 5, comprising (0.5 ± 0.1) mol ethanol per mol *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide, as determined by ¹H-NMR.
(7) The crystalline solvate according to any one of items 3 to 6, characterized by a mass change of less than 0.5% w/w based on the weight of the crystalline solvate according to any one of items 3 to 6, as determined by gravimetric moisture sorption in the range of from 0 to 95% relative humidity at a temperature of (25.0 ± 0.1) °C.
(8) A process for the preparation of the crystalline solvate according to any one of items 3 to 7 comprising:
   (i) providing *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide in crystalline form, amorphous form or as a mixture of these forms;
   (ii) preparing a solution of *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide provided in (i) in a solvent comprising ethanol, wherein the acetone content of the solvent is not more than 10% v/v based on the amount of ethanol;
   (iii) subjecting the solution prepared in (ii) to crystallization conditions comprising cooling the solution to a temperature of 20 °C or below;
(9) The crystalline solvate according to item 1, wherein the organic solvent is n-propanol and which is characterized by having a powder X-ray diffractogram comprising reflections at 2-Theta angles of (7.4 ± 0.2)°, (11.2 ± 0.2)°, (15.2 ± 0.2)°, (16.3 ± 0.2)°, (26.3 ± 0.2)°, when measured at a temperature in the range of from 15 to 25 °C with Cu-Kalpha_{1,2} radiation having a wavelength of 0.15419 nm.
(10) The crystalline solvate according to item 9, having a Fourier transform infrared spectrum comprising peaks at wavenumbers of (1762 ± 2) cm⁻¹, (1696 ± 2) cm⁻¹, (1618 ± 2) cm⁻¹, (1520 ± 2) cm⁻¹, (1135 ± 2) cm⁻¹, when measured at a temperature in the range of from 15 to 25 °C with a ZnSe ATR cell.
(11) The crystalline solvate according to item 9 or 10, comprising (0.5 ± 0.1) mol n-propanol per mol *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide, as determined by ¹H-NMR.
(12) A process for the preparation of the crystalline solvate according to any one of items 9 to 11 comprising:
   (i) providing *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide in crystalline form, amorphous form or as a mixture of these forms;
   (ii) preparing a solution of *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide provided in (i) in a solvent comprising n-propanol;
   (iii) subjecting the solution prepared in (ii) to crystallization conditions comprising cooling the solution to a temperature of 20 °C or below;
(13) The crystalline solvate according to item 1, wherein the organic solvent is acetone and which is characterized by having a powder X-ray diffractogram comprising reflections at 2-Theta angles of (7.4 ± 0.2)°, (11.2 ± 0.2)°, (15.2 ± 0.2)°, (16.4 ± 0.2)° and (26.3 ± 0.2)°, when measured at a temperature in the range of from 15 to 25 °C with Cu-Kα_{1,2} radiation having a wavelength of 0.15419 nm.
(14) The crystalline solvate according to item 13, having a Fourier transform infrared spectrum comprising peaks at wavenumbers of (1762 ± 2) cm⁻¹, (1700 ± 2) cm⁻¹, (1618 ± 2) cm⁻¹, (1519 ± 2) cm⁻¹ and (1136 ± 2) cm⁻¹, when measured at a temperature in the range of from 15 to 25 °C with a ZnSe ATR cell.
(15) The crystalline solvate according to item 13 or 14, comprising (0.5 ± 0.1) mol acetone per mol *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide, as determined by ¹H-NMR.
(16) A process for the preparation of the crystalline solvate according to any one of items 13 to 15 comprising:
   (i) providing *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide in crystalline form, amorphous form or as a mixture of these forms;
   (ii) preparing a solution of *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide provided in (i) in a solvent comprising acetone;
   (iii) subjecting the solution prepared in (ii) to crystallization conditions comprising cooling the solution to a temperature of 20 °C or below and at least partially evaporating the solvent at said temperature;
(17) The crystalline solvate according to item 1, wherein the organic solvent is methylethyl ketone and which is characterized by having a powder X-ray diffractogram comprising reflections at 2-Theta angles of (7.4 ± 0.2)°, (11.2 ± 0.2)°, (15.1 ± 0.2)°, (16.3 ± 0.2)° and (26.2 ± 0.2)°, when measured at a temperature in the range of from 15 to 25 °C with Cu-Kα_{1,2} radiation having a wavelength of 0.15419 nm.
(18) The crystalline solvate according to item 17, having a Fourier transform infrared spectrum comprising peaks at wavenumbers of (1762 ± 2) cm⁻¹, (1695 ± 2) cm⁻¹, (1618 ± 2) cm⁻¹, (1519 ± 2) cm⁻¹ and (1136 ± 2) cm⁻¹, when measured at a temperature in the range of from 15 to 25 °C with a ZnSe ATR cell.
(19) The crystalline solvate according to item 17 or 18, comprising (0.5 ± 0.1) mol methylethyl ketone per mol *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide, as determined by ¹H-NMR.
(20) A process for the preparation of the crystalline methylethyl ketone solvate according to any one of items 17 to 19 comprising:
   (i) providing *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide in crystalline form, amorphous form or as a mixture of these forms;
   (ii) preparing a solution of *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide provided in (i) in a solvent comprising methylethyl ketone;
   (iii) subjecting the solution prepared in (ii) to crystallization conditions comprising cooling the solution to a temperature of 20 °C or below and at least partially evaporating the solvent at said temperature;
(21) The process according to any one of items 8, 12, 16 and 20 further comprising (iv) separating at least a portion of the crystals obtained in (iii) from their mother liquor.

As used herein, the term "mother liquor" refers to the solution remaining after crystallization of a solid.
(22) The process according to item 21 further comprising (v) drying the crystals obtained in (iv) at a temperature of 100 °C or below.
(23) Use of the crystalline form according to any one of items 1 to 7, 9 to 11, 13 to 15, 17 to 19 or mixtures thereof for the preparation of a pharmaceutical composition.
(24) A pharmaceutical composition comprising the crystalline solvate according to any one of items 1 to 7, 9 to 11, 13 to 15, 17 to 19 or mixtures thereof and at least one pharmaceutically acceptable excipient.
(25) The pharmaceutical composition of item 24 which is an oral dosage form.
(26) The pharmaceutical composition of item 24 or 25 which is a tablet or capsule.
(27) The crystalline solvate according to any one of items 1 to 7, 9 to 11, 13 to 15, 17 to 19, or mixtures thereof, for use as a medicament.
(28) The crystalline solvate according to any one of items 1 to 7, 9 to 11, 13 to 15, 17 to 19, or mixtures thereof, for use in the treatment or prevention of a disease selected from the group consisting of psoriatric arthritis, plaque psoriasis, ankylosing spondylitis, Behcet's disease, rheumatoid arthritis, chronic prostatitis, chronic pelvic pain syndrome, vulvodynia, Crohn's disease, ulcerative colitis.

### BRIEF DESCRIPTION OF THE FIGURES

- Figure 1:: Representative PXRD of the novel *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide ethanol solvate
- Figure 2:: Representative FTIR spectrum of the novel *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide ethanol solvate

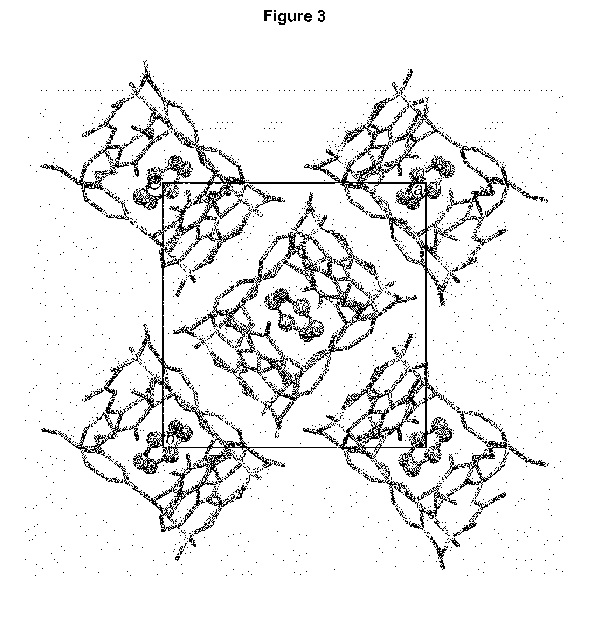

- Figure 3:: Crystal structure of the *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide ethanol solvate, viewed along the crystallographic *c* axis. The atoms of the main component are drawn in capped-sticks style and those of ethanol molecules are drawn as spheres. Hydrogen atoms and one disorder fragment of each ethanol position have been omitted for clarity.
- Figure 4:: Representative moisture sorption desorption isotherms of *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide ethanol solvate
- Figure 5:: Light microscopic image of the novel *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide ethanol solvate
- Figure 6:: Representative PXRD of the novel *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide n-propanol solvate
- Figure 7:: Representative FTIR spectrum of the novel *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide n-propanol solvate
- Figure 8:: Light microscopic image of the novel *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide n-propanol solvate
- Figure 9:: Representative PXRD of the novel *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide acetone solvate
- Figure 10:: Representative FTIR spectrum of the novel *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide acetone solvate
- Figure 11:: Representative PXRD of the novel *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide methylethyl ketone solvate
- Figure 12:: Representative FTIR spectrum of the novel *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide methylethyl ketone solvate

### DETAILED DESCRIPTION OF THE INVENTION

In the following, the present invention is described in more detail while referring to preferred embodiments and examples, which are presented however for illustrative purposes and shall not be construed to limit the invention in any way.

In the context of the present invention, the following abbreviations have the indicated meaning, unless explicitly stated otherwise:
- PXRD: powder X-ray diffraction/ diffractogram
- FTIR: Fourier transform infrared
- RT: room temperature
- RH: relative humidity
- microm: micrometer
- ICH: International Conference on Harmonization
- v: volume
- w: weight

As used herein the term "room temperature" indicates that the applied temperature is not critical and that no exact temperature value has to be kept. Usually, "room temperature" is understood to mean temperatures in the range of from 15 to 25 °C [see e.g. European Pharmacopoeia 8.2, 1.2 (2014)].

The term "crystalline solvate" as used herein refers to a solid where an organic solvent is coordinated in or accommodated by the crystal structure.

The term "ICH class 3 solvent" as used herein refers to solvents listed in table 3 of ICH HARMONIZED TRIPARTITE GUIDELINE, impurities: guideline for residual solvents Q3C(R5), current step 4 version, February 4th, 2011. These solvents are classified as solvents with low toxic potential and it is considered that amounts of these solvents of 50 mg per day or less would be acceptable without justification.

The term "essentially the same" with reference to PXRD means that variabilities in positions and relative intensities of reflections are to be taken into account. For example, a typical precision of the 2-Theta values is in the range of ± 0.2° 2-Theta. Thus, a reflection that usually appears at 7.4° 2-Theta for example can appear between 7.2° and 7.6° 2-Theta on most X-ray diffractometers under standard conditions. Furthermore, one skilled in the art will appreciate that relative intensities of the reflections will show inter-apparatus variability as well as variability due to degree of crystallinity, preferred orientation, sample preparation and other factors known to those skilled in the art and should be taken as qualitative measure only.

The term "essentially the same" with reference to infrared spectrometry means that variabilities in peak positions and relative intensities of the peaks are to be taken into account. For example, a typical precision of the wavenumber values is in the range of ± 2 cm⁻¹. Thus, a peak at 1696 cm⁻¹ for example can appear between 1694 and 1698 cm⁻¹ on most IR-spectrometers under standard conditions. Differences in relative intensities are typically smaller compared to X-ray diffraction. However, one skilled in the art will appreciate that small differences in peak intensities due to degree of crystallinity, sample preparation and other factors can also occur in IR-spectroscopy. Relative peak intensities should therefore be taken as qualitative measure only.

The term "non-hygroscopic" as used herein refers to compounds showing a mass change of less than 2 weight% based on the weight of the compound in the range of from 0 to 95% relative humidity at (25.0 ± 0.1) °C.

A first aspect of the invention are novel crystalline solvates of *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide and an organic solvent, wherein the organic solvent is selected from an ICH class 3 solvent which has a water solubility of at least 10% w/w, preferably of at least 20% w/w, more preferably of at least 30% w/w and most preferably of about 100% w/w when measured at room temperature.

Preferably, the ICH class 3 solvent is selected from the group consisting of acetic acid, acetone, 2-butanol, dimethyl sulfoxide, ethanol, formic acid, methyl acetate, methylethyl ketone, n-propanol, isopropanol or mixtures thereof, more preferably the ICH class 3 solvent is selected from the group consisting of from acetone, ethanol, methylethyl ketone, n-propanol or mixtures thereof and most preferably the ICH class 3 solvent is ethanol.

The novel crystalline solvates of *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide can be prepared by a process comprising:
(i) providing *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide in crystalline form, amorphous form or as a mixture of these forms;
(ii) preparing a solution of the *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide provided in (i) in an organic solvent selected from an ICH class 3 solvent which has a water solubility of at least 10% w/w;
(iii) optionally filtering the solution;
(iv) subjecting the solution provided in (ii) or optionally in (iii) to crystallization conditions comprising cooling the solution to a temperature of 20 °C or below;
(v) optionally separating at least a portion of the *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide solvate crystals obtained in (iv) from their mother liquor;
(vi) optionally drying the crystals obtained in (v);

The *N*-[2-[(1*S*)-1-(3-ethoxy-4-methoxyphenyl)-2-(methylsulfonyl)ethyl]-2,3-dihydro-1,3-dioxo-1*H-*isoindol-4-yl]acetamide starting material applied in (i) can for example be prepared in accordance with the procedure disclosed in example 2 of WO 2003/080049 A1 or according to the procedures described in examples 12 of WO 2009/120167 A1.

In a specific embodiment, the present invention relates to a novel crystalline ethanol solvate of *N*-[2-[(1*S*)-1-(3-ethoxy-4-methoxyphenyl)-2-(methylsulfonyl)ethyl]-2,3-dihydro-1,3-dioxo-1*H-*isoindol-4-yl]acetamide hereinafter also designated "form S_{EtOH}".

The novel crystalline ethanol solvate of *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide may be characterized by analytical methods well known in the field of the pharmaceutical industry for characterizing solids. Such methods comprise but are not limited to powder X-ray diffraction, single crystal X-ray diffraction, Fourier transform infrared spectroscopy, thermogravimetric analysis, differential scanning calorimetry and gravimetric moisture sorption. The novel crystalline ethanol solvate of *N*-[2-[(1*S*)-1-(3-ethoxy-4-methoxyphenyl)-2-(methylsulfonyl)ethyl]-2,3-dihydro-1,3-dioxo-1*H-*isoindol-4-yl]acetamide may be characterized by one analytical method or by combining two or more analytical methods. In particular, the novel ethanol solvate of *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide may be characterized by any one of the following embodiments or by combining two or more of the following embodiments.

Preferably, the crystalline ethanol solvate of *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide is characterized by having a powder X-ray diffractogram comprising reflections at 2-Theta angles of (7.4 ± 0.2)°, (11.2 ± 0.2)°, (15.2 ± 0.2)°, (16.3 ± 0.2)°, (26.3 ± 0.2)°, when measured at a temperature in the range of from 15 to 25 °C with Cu-Kalpha_{1,2} radiation having a wavelength of 0.15419 nm.

Particularly, the crystalline ethanol solvate of *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide is characterized by having a powder X-ray diffractogram comprising additional reflections at one or more 2-Theta angles selected from the group of (7.2 ± 0.2)°, (9.0 ± 0.2)°, (9.6 ± 0.2)°, (11.9 ± 0.2)°, (13.1 ± 0.2)°, (13.7 ± 0.2)°, (17.3 ± 0.2)°, (17.6 ± 0.2)°, (17.8 ± 0.2)°, (19.3 ± 0.2)°, (20.2 ± 0.2)°, (20.6 ± 0.2)°, (21.4 ± 0.2)°, (22.3 ± 0.2)°, (22.7 ± 0.2)°, (23.3 ± 0.2)°, (24.7 ± 0.2)°, (25.3 ± 0.2)°, (26.3 ± 0.2)°, (27.4 ± 0.2)°, (28.9 ± 0.2)°, when measured at a temperature in the range of from 15 to 25 °C with Cu-Kalpha_{1,2} radiation having a wavelength of 0.15419 nm.

Alternatively, the crystalline ethanol solvate of *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide is characterized by having a powder X-ray diffractogram comprising reflections at five or more 2-Theta angles selected from the group of (7.2 ± 0.2)°, (7.4 ± 0.2)°, (9.0 ± 0.2)°, (9.6 ± 0.2)°, (11.2 ± 0.2)°, (11.9 ± 0.2)°, (13.1 ± 0.2)°, (13.7 ± 0.2)°, (15.2 ± 0.2)°, (16.3 ± 0.2)°, (17.3 ± 0.2)°, (17.6 ± 0.2)°, (17.8 ± 0.2)°, (19.3 ± 0.2)°, (20.2 ± 0.2)°, (20.6 ± 0.2)°, (21.4 ± 0.2)°, (22.3 ± 0.2)°, (22.7 ± 0.2)°, (23.3 ± 0.2)°, (24.7 ± 0.2)°, (25.3 ± 0.2)°, (26.3 ± 0.2)°, (27.4 ± 0.2)°, (28.9 ± 0.2)°, when measured at a temperature in the range of from 15 to 25 °C with Cu-Kalpha_{1,2} radiation having a wavelength of 0.15419 nm.

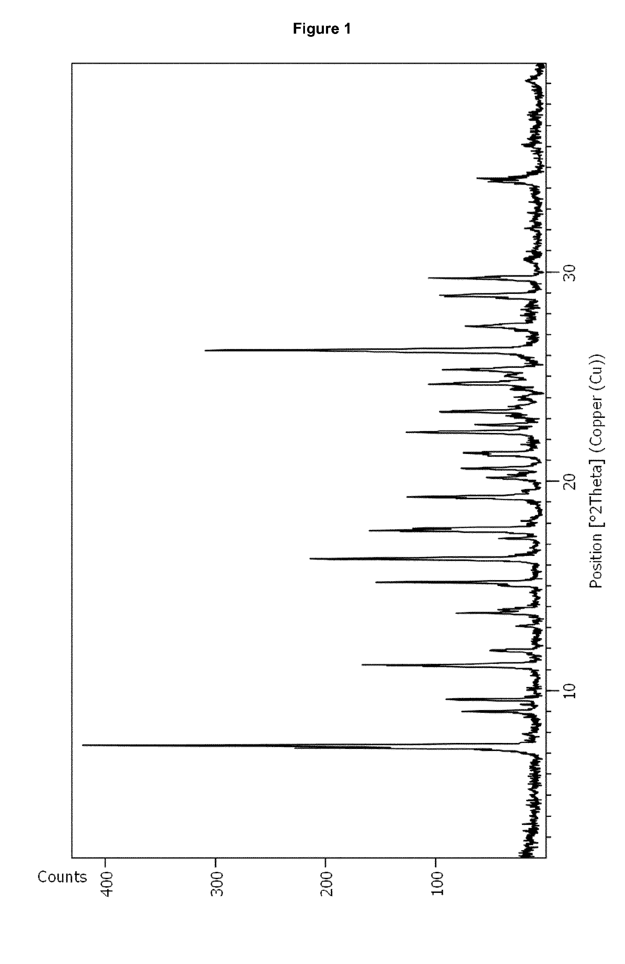

Alternatively or additionally, the crystalline ethanol solvate of *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide is characterized by having a powder X-ray diffractogram substantially the same as displayed in figure 1 of the present invention, when measured at a temperature in the range of from 15 to 25 °C with Cu-Kalpha_{1,2} radiation having a wavelength of 0.15419 nm.

Alternatively or additionally, the crystalline ethanol solvate of *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide is characterized by having a Fourier transform infrared spectrum comprising peaks at wavenumbers of (1762 ± 2) cm⁻¹, (1696 ± 2) cm⁻¹, (1618 ± 2) cm⁻¹, (1519 ± 2) cm⁻¹, (1135 ± 2) cm⁻¹, when measured at a temperature in the range of from 15 to 25 °C with a ZnSe ATR cell.

Particularly, the crystalline ethanol solvate of *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide is characterized by having a Fourier transform infrared spectrum comprising additional peaks at one or more wavenumbers selected from the group of (3370 ± 2) cm⁻¹, (2987 ± 2) cm⁻¹, (1592 ± 2) cm⁻¹, (1480 ± 2) cm⁻¹, (1394 ± 2) cm⁻¹, (1337 ± 2) cm⁻¹, (1299 ± 2) cm⁻¹, (1269 ± 2) cm⁻¹, (1101 ± 2) cm⁻¹, (1027 ± 2) cm⁻¹, (969 ± 2) cm⁻¹, (944± 2) cm⁻¹, (895 ± 2) cm⁻¹, (867 ± 2) cm⁻¹, (838 ± 2) cm^{- 1}, (823 ± 2) cm⁻¹, (753 ± 2) cm⁻¹, (741 ± 2) cm⁻¹, (655 ± 2) cm⁻¹, when measured at a temperature in the range of from 15 to 25 °C with a ZnSe ATR cell.

Alternatively, the crystalline ethanol solvate of *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide is characterized by having a Fourier transform infrared spectrum comprising peaks at five or more wavenumbers selected from the group of (3370 ± 2) cm⁻¹, (2987 ± 2) cm⁻¹, (1762 ± 2) cm⁻¹, (1696 ± 2) cm⁻¹, (1618 ± 2) cm⁻¹, (1592 ± 2) cm⁻¹, (1519 ± 2) cm⁻¹, (1480 ± 2) cm⁻¹, (1394 ± 2) cm⁻¹, (1337 ± 2) cm⁻¹, (1299 ± 2) cm⁻¹, (1269 ± 2) cm⁻¹, (1135 ± 2) cm⁻¹, (1101 ± 2) cm⁻¹, (1027 ± 2) cm⁻¹, (969 ± 2) cm⁻¹, (944 ± 2) cm⁻¹, (895 ± 2) cm⁻¹, (867 ± 2) cm⁻¹, (838 ± 2) cm⁻¹, (823 ± 2) cm⁻¹, (753 ± 2) cm⁻¹, (741 ± 2) cm⁻¹, (655 ± 2) cm⁻¹, when measured at a temperature in the range of from 15 to 25 °C with a ZnSe ATR cell.

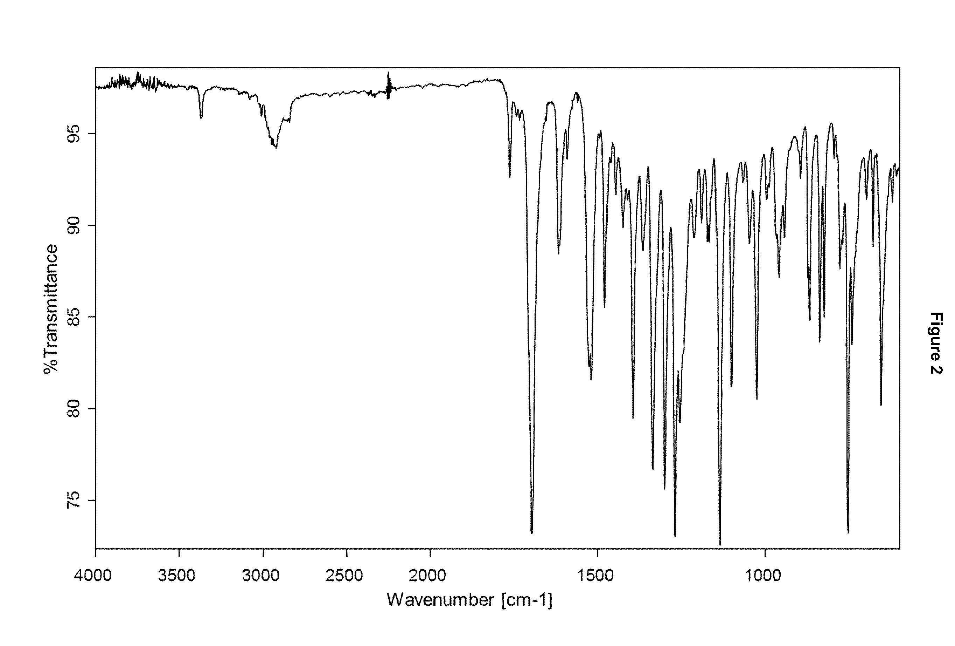

Alternatively or additionally, the crystalline ethanol solvate of *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide is characterized by having a Fourier transform infrared spectrum substantially the same as displayed in figure 2 of the present invention, when measured at a temperature in the range of from 15 to 25 °C with a ZnSe ATR cell.

Alternatively or additionally, the crystalline ethanol solvate of *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide is characterized by having the tetragonal space group symmetry P4₁2₁2 and the following unit cell parameters as determined by an X-ray single crystal structure analysis at 293 K:
a = (13.06 ± 0.04) Angstrom;
b = (13.06 ± 0.04) Angstrom;
c = (29.62 ± 0.09) Angstrom;
alpha = 90.0°;
beta = 90.0°;
gamma = 90.0°.

Alternatively or additionally, the crystalline ethanol solvate of *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide is characterized by having a crystal structure comprising 0.5 molecules ethanol per molecule *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide as determined by an X-ray single crystal structure analysis at 293 K.

Alternatively or additionally, the novel crystalline ethanol solvate of *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide is characterized by comprising 0.4 to 0.6 mol ethanol, preferably 0.5 mol ethanol such as (0.5 ± 0.1) mol ethanol per mol *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide as determined by ¹H-NMR.

Moreover, the novel crystalline ethanol solvate of *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide is a hemi ethanol solvate as determined by an X-ray single crystal structure analysis at 293 K and/or by ¹H-NMR.

Alternatively or additionally, the novel crystalline ethanol solvate of *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide is characterized by a mass change of less than 0.5% w/w based on the weight of the crystalline ethanol solvate of *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide as determined by gravimetric moisture sorption in the range of from 0 to 95% relative humidity at a temperature of (25.0 ± 0.1) °C.

Furthermore, the novel crystalline ethanol solvate of *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide is characterized as being non-hygroscopic as determined by gravimetric moisture sorption in the range of from 0 to 95% relative humidity at a temperature of (25.0 ± 0.1) °C.

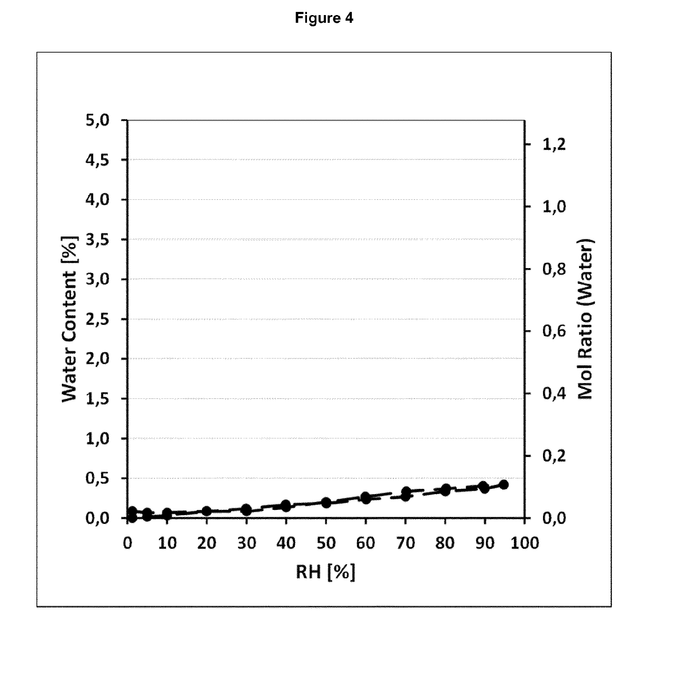

In addition, the crystalline ethanol solvate of *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide is characterized by having gravimetric moisture sorption desorption isotherms substantially the same as displayed in figure 4 of the present invention, when measured in the range of from 0 to 95% relative humidity at a temperature of (25.0 ± 0.1) °C.

Furthermore, the crystalline ethanol solvate of *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide is characterized by comprising particles having bipyramidal morphology.

The novel crystalline ethanol solvate of *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide can be prepared by a process comprising:
(i) providing *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide in crystalline form, amorphous form or as a mixture of these forms;
(ii) preparing a solution of the *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide provided in (i) in a solvent comprising ethanol, wherein the acetone content of the solvent is not more than 10% v/v based on the amount of ethanol;
(iii) optionally filtering the solution;
(iv) subjecting the solution provided in (ii) or optionally in (iii) to crystallization conditions comprising cooling the solution to a temperature of 20 °C or below;
(v) optionally separating at least a portion of the *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide ethanol solvate crystals obtained in (iv) from their mother liquor;
(vi) optionally drying the crystals obtained in (v);

The *N*-[2-[(1*S*)-1-(3-ethoxy-4-methoxyphenyl)-2-(methylsulfonyl)ethyl]-2,3-dihydro-1,3-dioxo-1*H-*isoindol-4-yl]acetamide starting material applied in (i) can for example be prepared in accordance with the procedure disclosed in example 2 of WO 2003/080049 A1 or according to the procedures described in examples 12 of WO 2009/120167 A1.

The solvent applied in (ii) comprises not more than 10% v/v, preferably not more than 5% v/v and more preferably not more than 1% v/v of acetone based on the amount of ethanol. Most preferably, the solvent is substantially free of acetone. In a preferred embodiment, the solvent applied in (ii) comprises not more than 10% v/v, preferably not more than 5% v/v and more preferably not more than 1% v/v of additional organic solvents based on the amount of ethanol. Most preferably, ethanol is the only organic solvent applied.

Preferably, the solvent provided in (ii) comprises aqueous ethanol, whereat the ethanol content preferably is in the range of from about 25 to 99% v/v, more preferably from about 25 to 75% v/v and most preferably from about 25 to 50% v/v. In addition, the water content of the solvent provided in (ii) preferably is in the range of from about 1 to 75% v/v, more preferably from about 25 to 75% v/v and most preferably from about 50 to 75% v/v.

Furthermore, the *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide concentration of the solution prepared in (ii) is in the range of from about 0.5 to 25 g per liter solvent, preferably from about 1 to 15 g per liter solvent, more preferably from about 1.5 to 10 g per liter solvent and most preferably from about 2 to 5 g per liter solvent.

The solution in (ii) may be prepared by increasing the temperature of the initial mixture comprising *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide and the applied solvent to a temperature of about 50 °C or above, preferably of about 60 °C or above, more preferably of about 70 °C or above and most preferably the mixture is heated to reflux temperature.

Once a solution is obtained, an optional filtration step may be applied in order to remove any possible undissolved particles. Optionally, the solution may be treated with charcoal before filtration.

In (iv) the solution obtained in (ii) or optionally in (iii) is subjected to crystallization conditions comprising cooling the solution to a temperature of about 20 °C or below, preferably of about 15 °C or below, more preferably of about 10 °C or below and most preferably of about 5 °C or below. The cooling rate is not critical with regards to the building of the ethanol solvate. The solution is kept at the applied temperature for about 1 to 14 days, preferably for about 1 to 7 days, more preferably for about 1 to 3 days and most preferably for about 1 to 2 days. In a preferred embodiment, the solution is not subjected to any agitation over this period.

Subsequently, the obtained *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide ethanol solvate crystals may be separated from their mother liquor by any conventional method such as filtration, centrifugation, evaporation of the solvent, decantation of the solvent or by two or more of these methods. Most preferably the *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide ethanol solvate crystals are collected by filtration.

Finally, the isolated *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide ethanol solvate crystals may be dried at a temperature of about 100 °C or less, preferably of about 80 °C or less, more preferably of about 60 °C or less and most preferably the crystals are dried at a temperature of about 40 °C or less for example at about room temperature. Drying may be performed at ambient pressure and/ or by applying vacuum. Drying is carried out at an atmosphere having a pressure of about 1013 mbar or less, preferably of about 100 mbar or less, more preferably of about 50 mbar or less and most preferably of about 30 mbar or less. Drying may be performed for a period of about 168 hours or less, preferably of about 84 hours or less, more preferably of about 48 hours or less and most preferably of about 24 hours or less.

In a further specific embodiment, the present invention relates to a novel crystalline n-propanol solvate of *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide hereinafter also designated "form S_{n-PrOH}".

The novel crystalline n-propanol solvate of *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide may be characterized by analytical methods well known in the field of the pharmaceutical industry for characterizing solids. Such methods comprise but are not limited to powder X-ray diffraction, single crystal X-ray diffraction, Fourier transform infrared spectroscopy, thermogravimetric analysis, differential scanning calorimetry and gravimetric moisture sorption. The novel crystalline n-propanol solvate of *N*-[2-[(1*S*)-1-(3-ethoxy-4-methoxyphenyl)-2-(methylsulfonyl)ethyl]-2,3-dihydro-1,3-dioxo-1*H-*isoindol-4-yl]acetamide may be characterized by one analytical method or by combining two or more analytical methods. In particular, the novel n-propanol solvate of *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide may be characterized by any one of the following embodiments or by combining two or more of the following embodiments.

Preferably, the crystalline n-propanol solvate of *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide is characterized by having a powder X-ray diffractogram comprising reflections at 2-Theta angles of (7.4 ± 0.2)°, (11.2 ± 0.2)°, (15.2 ± 0.2)°, (16.3 ± 0.2)°, (26.3 ± 0.2)°, when measured at a temperature in the range of from 15 to 25 °C with Cu-Kalpha_{1,2} radiation having a wavelength of 0.15419 nm.

Particularly, the crystalline n-propanol solvate of *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide is characterized by having a powder X-ray diffractogram comprising additional reflections at one or more 2-Theta angles selected from the group of (7.3 ± 0.2)°, (9.0 ± 0.2)°, (9.6 ± 0.2)°, (11.9 ± 0.2)°, (13.6 ± 0.2)°, (13.9 ± 0.2)°, (17.6 ± 0.2)°, (17.7 ± 0.2)°, (19.3 ± 0.2)°, (20.2 ± 0.2)°, (20.7 ± 0.2)°, (21.3 ± 0.2)°, (22.4 ± 0.2)°, (23.4 ± 0.2)°, (24.7 ± 0.2)°, (25.4 ± 0.2)°, (27.4 ± 0.2)°, (28.2 ± 0.2)°, (28.9 ± 0.2)°, (29.7 ± 0.2)°, when measured at a temperature in the range of from 15 to 25 °C with Cu-Kalpha_{1,2} radiation having a wavelength of 0.15419 nm.

Alternatively, the crystalline n-propanol solvate of *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide is characterized by having a powder X-ray diffractogram comprising reflections at five or more 2-Theta angles selected from the group of (7.3 ± 0.2)°, (7.4 ± 0.2)°, (9.0 ± 0.2)°, (9.6 ± 0.2)°, (11.2 ± 0.2)°, (11.9 ± 0.2)°, (13.6 ± 0.2)°, (13.9 ± 0.2)°, (15.2 ± 0.2)°, (16.3 ± 0.2)°, (17.7 ± 0.2)°, (19.3 ± 0.2)°, (20.2 ± 0.2)°, (20.7 ± 0.2)°, (21.3 ± 0.2)°, (22.4 ± 0.2)°, (23.4 ± 0.2)°, (24.7 ± 0.2)°, (25.4 ± 0.2)°, (26.3 ± 0.2)°, (27.4 ± 0.2)°, (28.2 ± 0.2)°, (28.9 ± 0.2)°, (29.7 ± 0.2)°, when measured at a temperature in the range of from 15 to 25 °C with Cu-Kalpha_{1,2} radiation having a wavelength of 0.15419 nm.

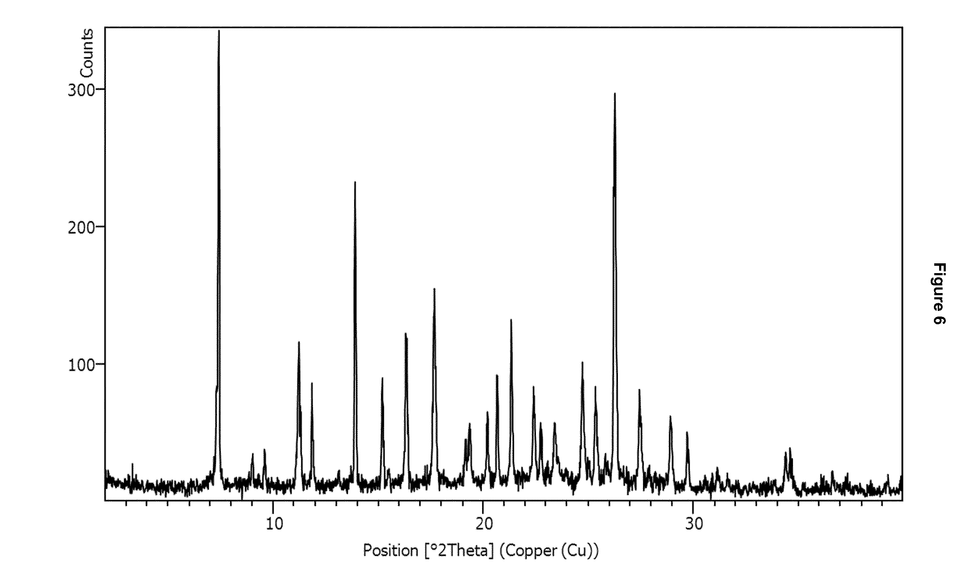

Moreover, the crystalline n-propanol solvate of *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide is characterized by having a powder X-ray diffractogram substantially the same as displayed in figure 6 of the present invention, when measured at a temperature in the range of from 15 to 25 °C with Cu-Kalpha_{1,2} radiation having a wavelength of 0.15419 nm.

Alternatively or additionally, the crystalline n-propanol solvate of *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide is characterized by having a Fourier transform infrared spectrum comprising peaks at wavenumbers of (1762 ± 2) cm⁻¹, (1696 ± 2) cm⁻¹, (1618 ± 2) cm⁻¹, (1520 ± 2) cm⁻¹, (1135 ± 2) cm⁻¹, when measured at a temperature in the range of from 15 to 25 °C with a ZnSe ATR cell. Particularly, the crystalline n-propanol solvate of *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide is characterized by having a Fourier transform infrared spectrum comprising additional peaks at one or more wavenumbers selected from the group of (3370 ± 2) cm⁻¹, (2987 ± 2) cm⁻¹, (1591 ± 2) cm⁻¹, (1480 ± 2) cm⁻¹, (1395 ± 2) cm⁻¹, (1337 ± 2) cm⁻¹, (1299 ± 2) cm⁻¹, (1270 ± 2) cm⁻¹, (1101 ± 2) cm⁻¹, (1027 ± 2) cm⁻¹, (996 ± 2) cm⁻¹, (959 ± 2) cm⁻¹, (944 ± 2) cm⁻¹, (896 ± 2) cm⁻¹, (867 ± 2) cm⁻¹, (838 ± 2) cm⁻¹, (824 ± 2) cm⁻¹, (753 ± 2) cm⁻¹, (741 ± 2) cm⁻¹, (655 ± 2) cm⁻¹, when measured at a temperature in the range of from 15 to 25 °C with a ZnSe ATR cell.

Alternatively, the crystalline n-propanol solvate of *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide is characterized by having a Fourier transform infrared spectrum comprising peaks at five or more wavenumbers selected from the group of (3370 ± 2) cm⁻¹, (2987 ± 2) cm⁻¹, (1762 ± 2) cm⁻¹, (1696 ± 2) cm⁻¹, (1618 ± 2) cm⁻¹, (1591 ± 2) cm⁻¹, (1520 ± 2) cm⁻¹, (1480 ± 2) cm⁻¹, (1395 ± 2) cm⁻¹, (1337 ± 2) cm⁻¹, (1299 ± 2) cm⁻¹, (1270 ± 2) cm⁻¹, (1135 ± 2) cm⁻¹, (1101 ± 2) cm⁻¹, (1027 ± 2) cm⁻¹, (996 ± 2) cm⁻¹, (959 ± 2) cm⁻¹, (944 ± 2) cm⁻¹, (896 ± 2) cm⁻¹, (867 ± 2) cm⁻¹, (838 ± 2) cm⁻¹, (824 ± 2) cm⁻¹, (753 ± 2) cm⁻¹, (741 ± 2) cm⁻¹, (655 ± 2) cm⁻¹, when measured at a temperature in the range of from 15 to 25 °C with a ZnSe ATR cell.

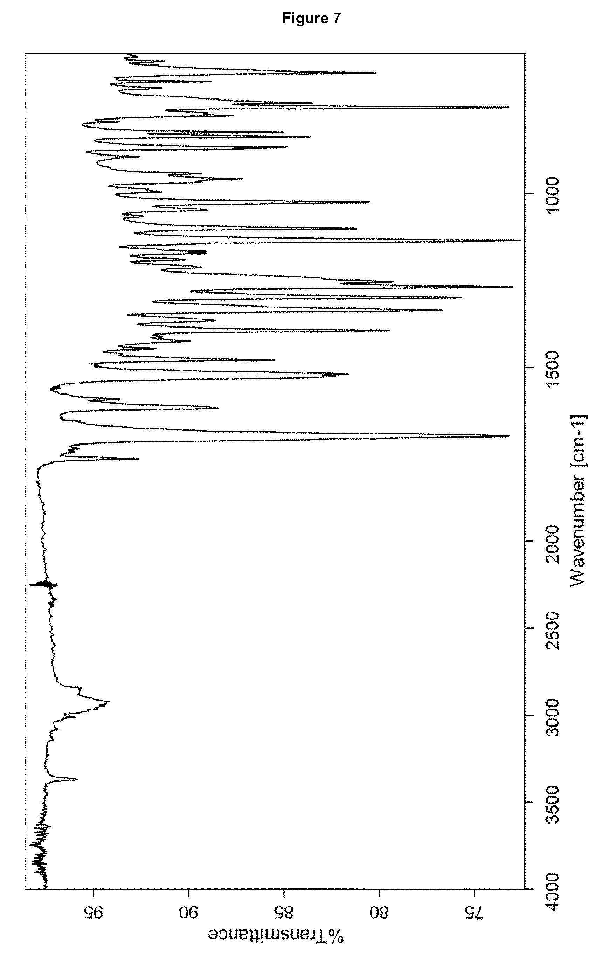

Moreover, the crystalline n-propanol solvate of *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide is characterized by having a Fourier transform infrared spectrum substantially the same as displayed in figure 7 of the present invention, when measured at a temperature in the range of from 15 to 25 °C with a ZnSe ATR cell.

PXRD and FTIR results show unequivocally that the n-propanol solvate is isostructural with the ethanol solvate. The crystal structures of these solvates contain one solvent-accessible void per formula unit whose center is intersected by a two-fold axis. The size of the void is such that only one solvent molecule can be accommodated at a time, resulting in 0.5 : 1.0 molar ratio of solvent and *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide.

Alternatively or additionally, the novel crystalline n-propanol solvate of *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide is characterized by comprising 0.4 to 0.6 mol n-propanol, preferably 0.5 mol n-propanol such as (0.5 ± 0.1) mol n-propanol per mol *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide as determined by ¹H-NMR.

Moreover, the novel crystalline n-propanol solvate of *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide is a hemi n-propanol solvate as determined by ¹H-NMR.

Furthermore, the crystalline n-propanol solvate of *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide is characterized by comprising particles having bipyramidal morphology.

The novel crystalline n-propanol solvate of *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide can be prepared by a process comprising:
(i) providing *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide in crystalline form, amorphous form or as a mixture of these forms;
(ii) preparing a solution of the *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide provided in (i) in a solvent comprising n-propanol;
(iii) optionally filtering the solution;
(iv) subjecting the solution provided in (ii) or optionally in (iii) to crystallization conditions comprising cooling the solution to a temperature of 20 °C or below;
(v) optionally separating at least a portion of the *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide n-propanol solvate crystals obtained in (iv) from their mother liquor;
(vi) optionally drying the crystals obtained in (v);

The *N*-[2-[(1*S*)-1-(3-ethoxy-4-methoxyphenyl)-2-(methylsulfonyl)ethyl]-2,3-dihydro-1,3-dioxo-1*H-*isoindol-4-yl]acetamide starting material applied in (i) may for example be prepared in accordance with the procedure disclosed in example 2 of WO 2003/080049 A1 or according to the procedures described in examples 12 of WO 2009/120167 A1.

The solvent applied in (ii) comprises not more than 10% v/v, preferably not more than 5% v/v and more preferably not more than 1% v/v of additional organic solvents based on the amount of n-propanol. Most preferably, n-propanol is the only organic solvent applied.

Preferably, the solvent provided in (ii) comprises aqueous n-propanol, whereat the n-propanol content preferably is in the range of from about 25 to 99% v/v, more preferably from about 25 to 75% v/v and most preferably from about 25 to 50% v/v. In addition, the water content of the solvent provided in (ii) preferably is in the range of from about 1 to 75% v/v, preferably from about 25 to 75% v/v, more preferably from about 50 to 75% v/v.

Furthermore, the *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide concentration of the solution prepared in (ii) is in the range of from about 0.5 to 25 g per liter solvent, preferably from about 1 to 15 g per liter solvent, more preferably from about 1.5 to 10 g per liter solvent and most preferably from about 2 to 5 g per liter solvent.

The solution in (ii) may be prepared by increasing the temperature of the initial mixture comprising *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide and the applied solvent to a temperature of about 50 °C or above, preferably of about 70 °C or above, more preferably of about 90 °C or above and most preferably the mixture is heated to reflux temperature.

Once a solution is obtained, an optional filtration step may be applied in order to remove any possible undissolved particles. Optionally, the solution may be treated with charcoal before filtration.

In (iv) the solution obtained in (ii) or optionally in (iii) is subjected to crystallization conditions comprising cooling the solution to a temperature of about 20 °C or below, preferably of about 15 °C or below, more preferably of about 10 °C or below and most preferably of about 5 °C or below. The cooling rate is not critical with regards to the building of the n-propanol solvate. The solution is kept at the applied temperature for about 1 to 14 days, preferably for about 1 to 7 days, more preferably for about 1 to 3 days and most preferably for about 1 to 2 days. In a preferred embodiment, the solution is not subjected to any agitation over this period.

Subsequently, the obtained *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide n-propanol solvate crystals may be separated from their mother liquor by any conventional method such as filtration, centrifugation, evaporation of the solvent, decantation of the solvent or by two or more of these methods. Most preferably the *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide n-propanol solvate crystals are collected by filtration.

Finally, the isolated *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide n-propanol solvate crystals may be dried at a temperature of about 100 °C or less, preferably of about 80 °C or less, more preferably of about 60 °C or less and most preferably the crystals are dried at a temperature of about 40 °C or less for example at about room temperature. Drying may be performed at ambient pressure and/or by applying vacuum. Drying is carried out at an atmosphere having a pressure of about 1013 mbar or less, preferably of about 100 mbar or less, more preferably of about 50 mbar or less and most preferably of about 30 mbar or less. Drying may be performed for a period of about 168 hours or less, preferably of about 84 hours or less, more preferably of about 48 hours or less and most preferably of about 24 hours or less.

In another specific embodiment, the present invention relates to a novel crystalline acetone solvate of *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide hereinafter also designated "form S_{ACT}".

The novel crystalline acetone solvate of *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide may be characterized by analytical methods well known in the field of the pharmaceutical industry for characterizing solids. Such methods comprise but are not limited to powder X-ray diffraction, single crystal X-ray diffraction, Fourier transform infrared spectroscopy, thermogravimetric analysis, differential scanning calorimetry and gravimetric moisture sorption. The novel crystalline acetone solvate of *N*-[2-[(1*S*)-1-(3-ethoxy-4-methoxyphenyl)-2-(methylsulfonyl)ethyl]-2,3-dihydro-1,3-dioxo-1*H-*isoindol-4-yl]acetamide may be characterized by one analytical method or by combining two or more analytical methods. In particular, the novel acetone solvate of *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide may be characterized by any one of the following embodiments or by combining two or more of the following embodiments.

Preferably, the crystalline acetone solvate of *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide is characterized by having a powder X-ray diffractogram comprising reflections at 2-Theta angles of (7.4 ± 0.2)°, (11.2 ± 0.2)°, (15.2 ± 0.2)°, (16.3 ± 0.2)°, (26.3 ± 0.2)°, when measured at a temperature in the range of from 15 to 25 °C with Cu-Kalpha_{1,2} radiation having a wavelength of 0.15419 nm.

Particularly, the crystalline acetone solvate of *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide is characterized by having a powder X-ray diffractogram comprising additional reflections at one or more 2-Theta angles selected from the group of (7.3 ± 0.2)°, (9.0 ± 0.2)°, (9.6 ± 0.2)°, (11.9 ± 0.2)°, (13.1 ± 0.2)°, (13.9 ± 0.2)°, (17.7 ± 0.2)°, (19.3 ± 0.2)°, (20.2 ± 0.2)°, (20.7 ± 0.2)°, (21.4 ± 0.2)°, (22.4 ± 0.2)°, (22.7 ± 0.2)°, (23.4 ± 0.2)°, (24.7 ± 0.2)°, (25.4 ± 0.2)°, (27.5 ± 0.2)°, (29.0 ± 0.2)°, (29.8 ± 0.2)°, when measured at a temperature in the range of from 15 to 25 °C with Cu-Kalpha_{1,2} radiation having a wavelength of 0.15419 nm.

Alternatively, the crystalline acetone solvate of *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide is characterized by having a powder X-ray diffractogram comprising reflections at five or more 2-Theta angles selected from the group of (7.3 ± 0.2)°, (7.4 ± 0.2)°, (9.0 ± 0.2)°, (9.6 ± 0.2)°, (11.2 ± 0.2)°, (11.9 ± 0.2)°, (13.1 ± 0.2)°, (13.9 ± 0.2)°, (15.2 ± 0.2)°, (16.4 ± 0.2)°, (17.7 ± 0.2)°, (19.3 ± 0.2)°, (20.2 ± 0.2)°, (20.7 ± 0.2)°, (21.4 ± 0.2)°, (22.4 ± 0.2)°, (22.7 ± 0.2)°, (23.4 ± 0.2)°, (24.7 ± 0.2)°, (25.4 ± 0.2)°, (26.3 ± 0.2)°, (27.5 ± 0.2)°, (29.0 ± 0.2)°, (29.8 ± 0.2)°, when measured at a temperature in the range of from 15 to 25 °C with Cu-Kalpha_{1,2} radiation having a wavelength of 0.15419 nm.

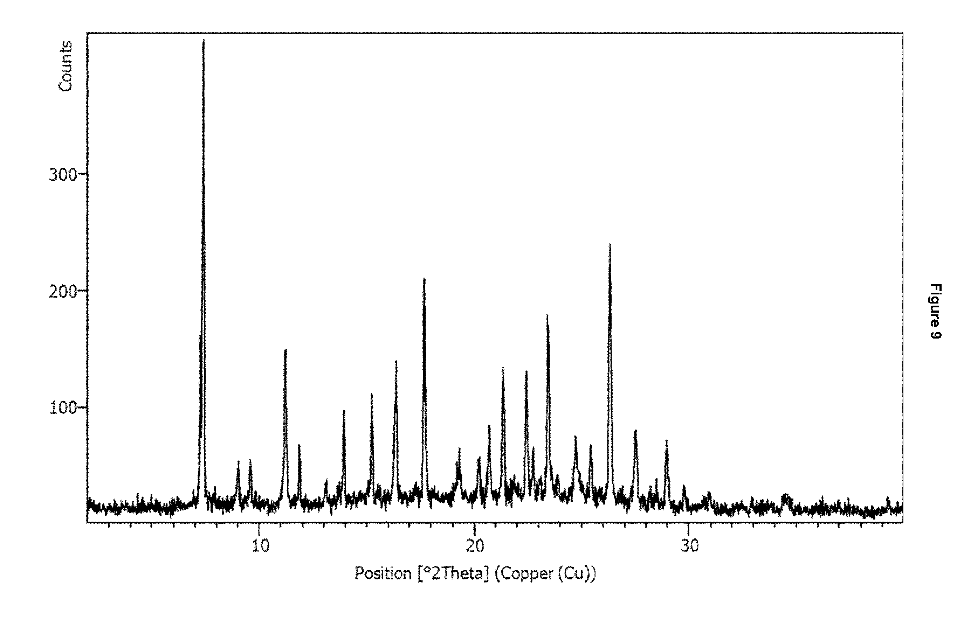

Moreover, the crystalline acetone solvate of *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide is characterized by having a powder X-ray diffractogram substantially the same as displayed in figure 9 of the present invention, when measured at a temperature in the range of from 15 to 25 °C with Cu-Kalpha_{1,2} radiation having a wavelength of 0.15419 nm.

Alternatively or additionally, the crystalline acetone solvate of *N*-[2-[(1*S*)-1-(3-ethoxy-4-methoxyphenyl)-2-(methylsulfonyl)ethyl]-2,3-dihydro-1,3-dioxo-1H-isoindol-4-yl]acetamide is characterized by having a Fourier transform infrared spectrum comprising peaks at wavenumbers of (1762 ± 2) cm⁻¹, (1700 ± 2) cm⁻¹, (1618 ± 2) cm⁻¹, (1519 ± 2) cm⁻¹, (1137 ± 2) cm⁻¹, when measured at a temperature in the range of from 15 to 25 °C with a ZnSe ATR cell.

Particularly, the crystalline acetone solvate of *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide is characterized by having a Fourier transform infrared spectrum comprising additional peaks at one or more wavenumbers selected from the group of (3369 ± 2) cm⁻¹, (2990 ± 2) cm⁻¹, (1592 ± 2) cm⁻¹, (1480 ± 2) cm⁻¹, (1395 ± 2) cm⁻¹, (1337 ± 2) cm⁻¹, (1299 ± 2) cm⁻¹, (1270 ± 2) cm⁻¹, (1101 ± 2) cm⁻¹, (1027 ± 2) cm⁻¹, (969 ± 2) cm⁻¹, (944 ± 2) cm⁻¹, (896 ± 2) cm⁻¹, (868 ± 2) cm⁻¹, (838 ± 2) cm⁻¹, (824 ± 2) cm⁻¹, (753 ± 2) cm⁻¹, (741 ± 2) cm⁻¹, (655 ± 2) cm⁻¹, when measured at a temperature in the range of from 15 to 25 °C with a ZnSe ATR cell.

Alternatively, the crystalline acetone solvate of *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide is characterized by having a Fourier transform infrared spectrum comprising peaks at five or more wavenumbers selected from the group of (3369 ± 2) cm⁻¹, (2990 ± 2) cm⁻¹, (1762 ± 2) cm⁻¹, (1700 ± 2) cm⁻¹, (1618 ± 2) cm⁻¹, (1592 ± 2) cm⁻¹, (1519 ± 2) cm⁻¹, (1480 ± 2) cm⁻¹, (1395 ± 2) cm⁻¹, (1337 ± 2) cm⁻¹, (1299 ± 2) cm⁻¹, (1270 ± 2) cm⁻¹, (1136 ± 2) cm⁻¹, (1101 ± 2) cm⁻¹, (1027 ± 2) cm⁻¹, (969 ± 2) cm⁻¹, (944 ± 2) cm⁻¹, (896 ± 2) cm⁻¹, (868 ± 2) cm⁻¹, (838 ± 2) cm⁻¹, (824 ± 2) cm⁻¹, (753 ± 2) cm⁻¹, (741 ± 2) cm⁻¹, (655 ± 2) cm⁻¹, when measured at a temperature in the range of from 15 to 25 °C with a ZnSe ATR cell.

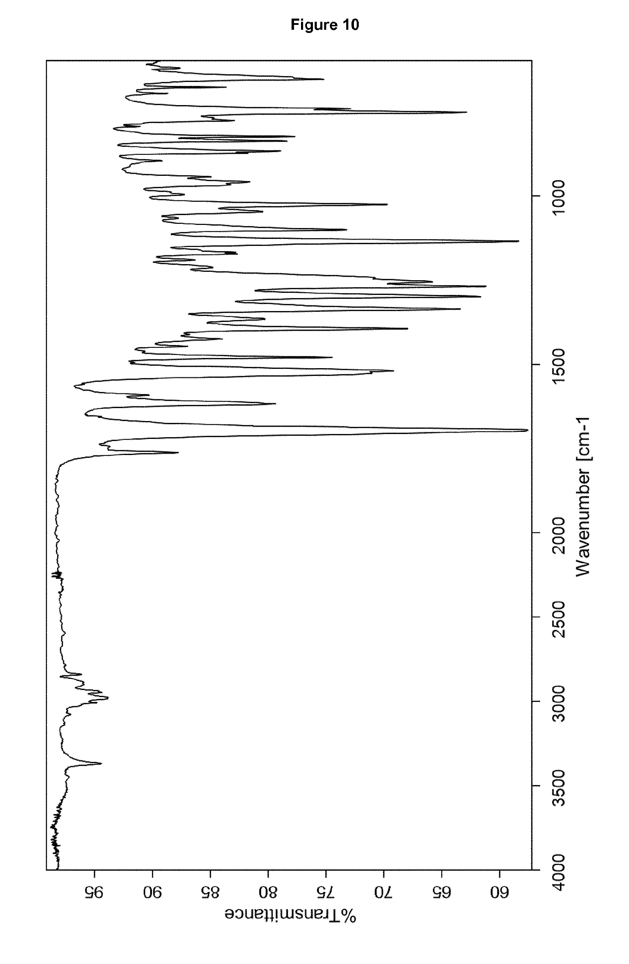

Moreover, the crystalline acetone solvate of *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide is characterized by having a Fourier transform infrared spectrum substantially the same as displayed in figure 10 of the present invention, when measured at a temperature in the range of from 15 to 25 °C with a ZnSe ATR cell.

PXRD and FTIR results show unequivocally that the acetone solvate is isostructural with the ethanol solvate. The crystal structures of these solvates contain one solvent-accessible void per formula unit whose center is intersected by a two-fold axis. The size of the void is such that only one solvent molecule can be accommodated at a time, resulting in 0.5 : 1.0 molar ratio of solvent and *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide.

Alternatively or additionally, the novel crystalline acetone solvate of *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide is characterized by comprising 0.4 to 0.6 mol acetone, preferably 0.5 mol acetone such as (0.5 ± 0.1) mol acetone per mol *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide as determined by ¹H-NMR.

Moreover, the novel crystalline acetone solvate of *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide is a hemi acetone solvate as determined by ¹H-NMR.

The novel crystalline acetone solvate of *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide can be prepared by a process comprising:
(i) providing *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide in crystalline form, amorphous form or as a mixture of these forms;
(ii) preparing a solution of the *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide provided in (i) in a solvent comprising acetone;
(iii) optionally filtering the solution;
(iv) subjecting the solution provided in (ii) or optionally in (iii) to crystallization conditions comprising cooling the solution to a temperature of 20 °C or below and at least partially evaporating the solvent at said temperature;
(v) optionally separating at least a portion of the *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide acetone solvate crystals obtained in (iv) from their mother liquor;
(vi) optionally drying the crystals obtained in (iv) or (v);

The *N*-[2-[(1*S*)-1-(3-ethoxy-4-methoxyphenyl)-2-(methylsulfonyl)ethyl]-2,3-dihydro-1,3-dioxo-1*H-*isoindol-4-yl]acetamide starting material applied in (i) may for example be prepared in accordance with the procedure disclosed in example 2 of WO 2003/080049 A1 or according to the general procedures described in examples 12 of WO 2009/120167 A1.

The solvent applied in (ii) comprises not more than 10% v/v, preferably not more than 5% v/v and more preferably not more than 1% v/v of additional organic solvents based on the amount of acetone. Most preferably, acetone is the only organic solvent applied.

Preferably, the solvent provided in (ii) comprises aqueous acetone, whereat the acetone content preferably is in the range of from about 25 to 99% v/v, more preferably from about 25 to 75% v/v and most preferably from about 25 to 50% v/v. In addition, the water content of the solvent provided in (ii) preferably is in the range of from about 1 to 75% v/v, preferably from about 25 to 75% v/v, more preferably from about 50 to 75% v/v.

Furthermore, the *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide concentration of the solution prepared in (ii) is in the range of from about 0.5 to 25 g per liter solvent, preferably from about 1 to 15 g per liter solvent, more preferably from about 1.5 to 10 g per liter solvent and most preferably from about 2 to 5 g per liter solvent.

The solution in (ii) may be prepared by increasing the temperature of the initial mixture comprising *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide and the applied solvent to a temperature of about 50 °C or above, preferably of about 40 °C or above, more preferably of about 30 °C or above and most preferably the solution is prepared at about room temperature.

Once a solution is obtained, an optional filtration step may be applied in order to remove any possible undissolved particles. Optionally, the solution may be treated with charcoal before filtration.

In (iv) the solution obtained in (ii) or optionally in (iii) is subjected to crystallization conditions comprising cooling the solution to a temperature of about 20 °C or less, preferably of about 15 °C or less, more preferably of about 10 °C or less and most preferably of about 5 °C or less and at least partially evaporating the applied solvent at said temperature. In a preferred embodiment, the solvent is evaporated at said temperature, more preferably the solvent is completely evaporated at said temperature.

The solution is evaporated at the applied temperature over a period of about 1 hour to 14 days, preferably of about 1 hour to 7 days, more preferably of about 1 hour to 1 day and most preferably of about 1 to 12 hours, whereat preferably no agitation is applied over this period.

Subsequently, the obtained *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide acetone solvate crystals are collected or separated from their mother liquor by any conventional method such as filtration, centrifugation, evaporation of the solvent, decantation of the solvent or by two or more of these methods. Finally, the isolated *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide acetone solvate crystals may be dried at a temperature of about 100 °C or less, preferably of about 80 °C or less, more preferably of about 60 °C or less and most preferably the crystals are dried at a temperature of about 40 °C or less for example at about room temperature. Drying may be performed at ambient pressure and/or by applying vacuum. Drying is carried out at an atmosphere having a pressure of about 1013 mbar or less, preferably of about 100 mbar or less, more preferably of about 50 mbar or less and most preferably of about 30 mbar or less. Drying may be performed for a period of about 168 hours or less, preferably for about 84 hours or less, more preferably for about 48 hours or less and most preferably for about 24 hours or less.

In a further specific embodiment, the present invention relates to a novel crystalline methylethyl ketone solvate of *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide hereinafter also designated "form S_{MEK}".

The novel crystalline methylethyl ketone solvate of *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide may be characterized by analytical methods well known in the field of the pharmaceutical industry for characterizing solids. Such methods comprise but are not limited to powder X-ray diffraction, single crystal X-ray diffraction, Fourier transform infrared spectroscopy, thermogravimetric analysis, differential scanning calorimetry and gravimetric moisture sorption. The novel crystalline methylethyl ketone solvate of *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide may be characterized by one analytical method or by combining two or more analytical methods. In particular, the novel methylethyl ketone solvate of *N*-[2-[(1*S*)-1-(3-ethoxy-4-methoxyphenyl)-2-(methylsulfonyl)ethyl]-2,3-dihydro-1,3-dioxo-1*H-*isoindol-4-yl]acetamide may be characterized by any one of the following embodiments or by combining two or more of the following embodiments.

Preferably, the crystalline methylethyl ketone solvate of *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide is characterized by having a powder X-ray diffractogram comprising reflections at 2-Theta angles of (7.4 ± 0.2)°, (11.2 ± 0.2)°, (15.1 ± 0.2)°, (16.3 ± 0.2)°, (26.2 ± 0.2)°, when measured at a temperature in the range of from 15 to 25 °C with Cu-Kalpha_{1,2} radiation having a wavelength of 0.15419 nm.

Particularly, the crystalline methylethyl ketone solvate of *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide is characterized by having a powder X-ray diffractogram comprising additional reflections at one or more 2-Theta angles selected from the group of (9.0 ± 0.2)°, (11.9 ± 0.2)°, (17.8 ± 0.2)°, (19.3 ± 0.2)°, (20.1 ± 0.2)°, (21.3 ± 0.2)°, (22.4 ± 0.2)°, (24.7 ± 0.2)°, (25.3 ± 0.2)°, (27.4 ± 0.2)°, (28.9 ± 0.2)°, when measured at a temperature in the range of from 15 to 25 °C with Cu-Kalpha_{1,2} radiation having a wavelength of 0.15419 nm.

Alternatively, the crystalline methylethyl ketone solvate of *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide is characterized by having a powder X-ray diffractogram comprising reflections at five or more 2-Theta angles selected from the group of (7.4 ± 0.2)°, (9.0 ± 0.2)°, (11.2 ± 0.2)°, (11.9 ± 0.2)°, (15.1 ± 0.2)°, (16.3 ± 0.2)°, (17.8 ± 0.2)°, (19.3 ± 0.2)°, (20.1 ± 0.2)°, (21.3 ± 0.2)°, (22.4 ± 0.2)°, (24.7 ± 0.2)°, (25.3 ± 0.2)°, (26.2 ± 0.2)°, (27.4 ± 0.2)°, (28.9 ± 0.2)°, when measured at a temperature in the range of from 15 to 25 °C with Cu-Kalpha_{1,2} radiation having a wavelength of 0.15419 nm.

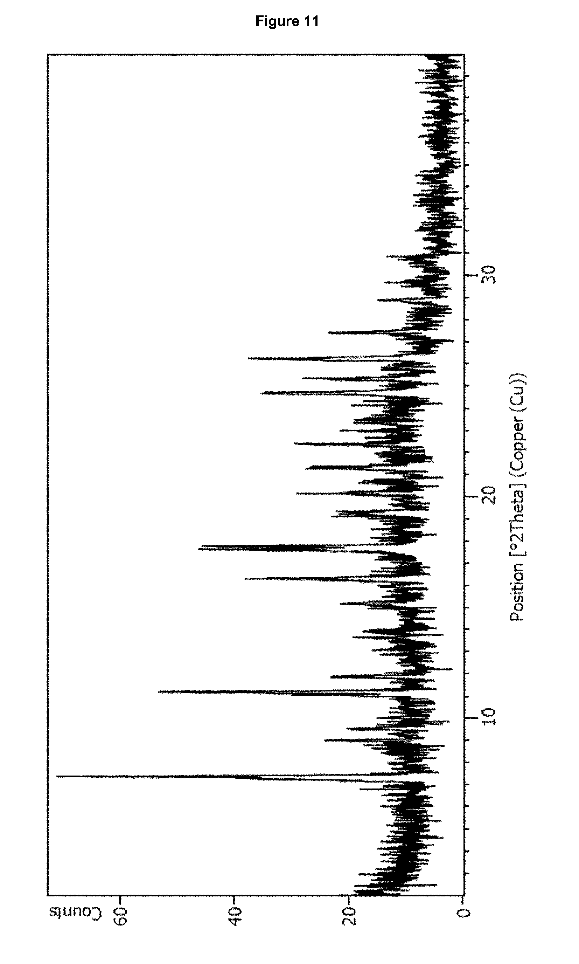

Moreover, the crystalline methylethyl ketone solvate of *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide is characterized by having a powder X-ray diffractogram substantially the same as displayed in figure 11 of the present invention, when measured at a temperature in the range of from 15 to 25 °C with Cu-Kalpha_{1,2} radiation having a wavelength of 0.15419 nm.

Alternatively or additionally, the crystalline methylethyl ketone solvate of *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide is characterized by having a Fourier transform infrared spectrum comprising peaks at wavenumbers of (1762 ± 2) cm⁻¹, (1695 ± 2) cm⁻¹, (1618 ± 2) cm⁻¹, (1519 ± 2) cm⁻¹, (1135 ± 2) cm⁻¹, when measured at a temperature in the range of from 15 to 25 °C with a ZnSe ATR cell.

Particularly, the crystalline methylethyl ketone solvate of *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide is characterized by having a Fourier transform infrared spectrum comprising additional peaks at one or more wavenumbers selected from the group of (3369 ± 2) cm⁻¹, (2978 ± 2) cm⁻¹, (1591 ± 2) cm⁻¹, (1480 ± 2) cm⁻¹, (1394 ± 2) cm⁻¹, (1336 ± 2) cm⁻¹, (1299 ± 2) cm⁻¹, (1269 ± 2) cm⁻¹, (1101 ± 2) cm⁻¹, (1026 ± 2) cm⁻¹, (997 ± 2) cm⁻¹, (960 ± 2) cm⁻¹, (896 ± 2) cm⁻¹, (867 ± 2) cm⁻¹, (838 ± 2) cm⁻¹, (824 ± 2) cm⁻¹, (752 ± 2) cm⁻¹, (655 ± 2) cm⁻¹, when measured at a temperature in the range of from 15 to 25 °C with a ZnSe ATR cell.

Alternatively, the crystalline methylethyl ketone solvate of *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide is characterized by having a Fourier transform infrared spectrum comprising peaks at five or more wavenumbers selected from the group of (3369 ± 2) cm⁻¹, (2978 ± 2) cm⁻¹, (1762 ± 2) cm⁻¹, (1695 ± 2) cm⁻¹, (1618 ± 2) cm⁻¹, (1591 ± 2) cm⁻¹, (1519 ± 2) cm⁻¹, (1480 ± 2) cm⁻¹, (1394 ± 2) cm⁻¹, (1336 ± 2) cm⁻¹, (1299 ± 2) cm⁻¹, (1269 ± 2) cm⁻¹, (1135 ± 2) cm⁻¹, (1101 ± 2) cm⁻¹, (1026 ± 2) cm⁻¹, (997 ± 2) cm⁻¹, (960 ± 2) cm⁻¹, (896 ± 2) cm⁻¹, (867 ± 2) cm⁻¹, (838 ± 2) cm⁻¹, (824 ± 2) cm⁻¹, (752 ± 2) cm⁻¹, (655 ± 2) cm⁻¹, when measured at a temperature in the range of from 15 to 25 °C with a ZnSe ATR cell.

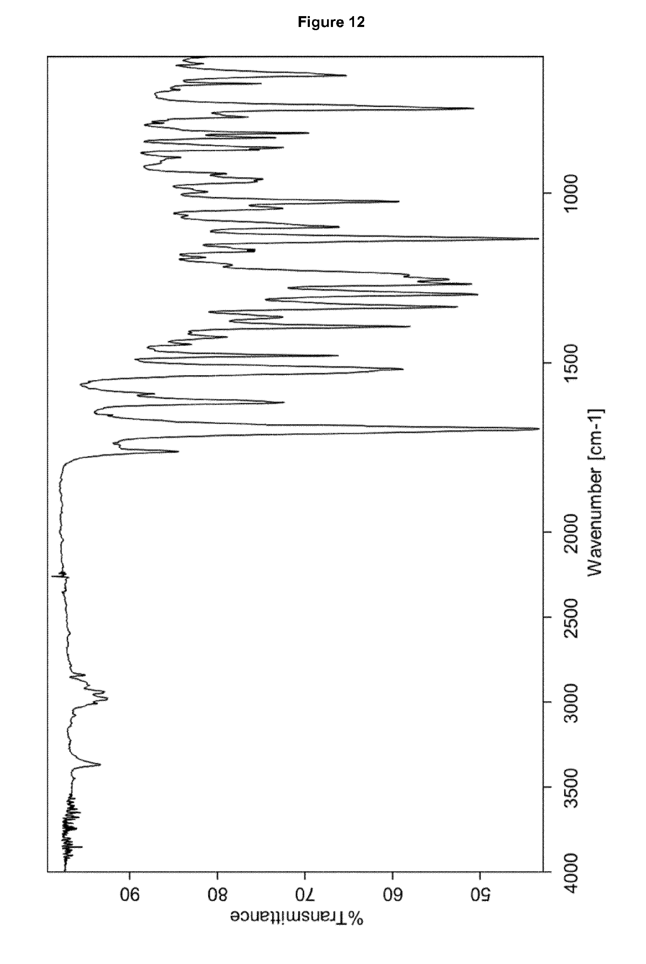

Moreover, the crystalline methylethyl ketone solvate of *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide is characterized by having a Fourier transform infrared spectrum substantially the same as displayed in figure 12 of the present invention, when measured at a temperature in the range of from 15 to 25 °C with a ZnSe ATR cell.

PXRD and FTIR results show unequivocally that the methylethyl ketone solvate is isostructural with the ethanol solvate. The crystal structures of these solvates contain one solvent-accessible void per formula unit whose center is intersected by a two-fold axis. The size of the void is such that only one solvent molecule can be accommodated at a time, resulting in 0.5 : 1.0 molar ratio of solvent and *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide.

Alternatively or additionally, the novel crystalline methylethyl ketone solvate of *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide is characterized by comprising 0.4 to 0.6 mol methylethyl ketone, preferably 0.5 mol methylethyl ketone such as (0.5 ± 0.1) mol methylethyl ketone per mol *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide as determined by ¹H-NMR.

Moreover, the novel crystalline methylethyl ketone solvate of *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide is a hemi methyl ethyl ketone solvate as determined by ¹H-NMR.

The novel crystalline methylethyl ketone solvate of *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide can be prepared by a process comprising:
(i) providing *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide in crystalline form, amorphous form or as a mixture of these forms;
(ii) preparing a solution of the *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide provided in (i) in a solvent comprising methylethyl ketone;
(iii) optionally filtering the solution;
(iv) subjecting the solution provided in (ii) or optionally in (iii) to crystallization conditions comprising cooling the solution to a temperature of 20 °C or below and at least partially evaporating the solvent at said temperature;
(v) optionally separating at least a portion of the *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide methylethyl ketone solvate crystals obtained in (iv) from their mother liquor;
(vi) optionally drying the crystals obtained in (iv) or (v);

The *N*-[2-[(1*S*)-1-(3-ethoxy-4-methoxyphenyl)-2-(methylsulfonyl)ethyl]-2,3-dihydro-1,3-dioxo-1*H-*isoindol-4-yl]acetamide starting material applied in (i) may for example be prepared in accordance with the procedure disclosed in example 2 of WO 2003/080049 A1 or according to the general procedures described in examples 12 of WO 2009/120167 A1.

The solvent applied in (ii) comprises not more than 10% v/v, preferably not more than 5% v/v and more preferably not more than 1% v/v of additional organic solvents based on the amount of methylethyl ketone. Most preferably, methylethyl ketone is the only organic solvent applied.

Preferably, the solvent provided in (ii) comprises aqueous methylethyl ketone, whereat the methylethyl ketone content preferably is in the range of from about 25 to 99% v/v, more preferably from about 25 to 75% v/v and most preferably from about 25 to 50% v/v. In addition, the water content of the solvent provided in (ii) preferably is in the range of from about 1 to 75% v/v, preferably from about 25 to 75% v/v, more preferably from about 50 to 75% v/v.

Furthermore, the *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide concentration of the solution prepared in (ii) is in the range of from about 0.5 to 25 g per liter solvent, preferably from about 1 to 15 g per liter solvent, more preferably from about 1.5 to 10 g per liter solvent and most preferably from about 2 to 5 g per liter solvent.

The solution in (ii) may be prepared by increasing the temperature of the initial mixture comprising *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide and the applied solvent to a temperature of about 50 °C or above, preferably of about 60 °C or above, more preferably of about 70 °C or above and most preferably the mixture is heated to reflux temperature.

Once a solution is obtained, an optional filtration step may be applied in order to remove any possible undissolved particles. Optionally, the solution may be treated with charcoal before filtration.

In (iv) the solution obtained in (ii) or optionally in (iii) is subjected to crystallization conditions comprising cooling the solution to a temperature of about 20 °C or less, preferably of about 15 °C or less, more preferably of about 10 °C or less and most preferably of about 5 °C or less and at least partially evaporating the applied solvent at said temperature. In a preferred embodiment, the solvent is evaporated at said temperature, more preferably the solvent is completely evaporated at said temperature.

The solution is evaporated at the applied temperature over a period of about 1 hour to 14 days, preferably of about 1 hour to 7 days, more preferably of about 1 hour to 1 day and most preferably of about 1 to 12 hours, whereat preferably no agitation is applied over this period.

Subsequently, the obtained *N*-[2-[(1S)-1-(3-ethoxy-4-methoxyphenyl)-2-(methylsulfonyl)ethyl]-2,3-dihydro-1,3-dioxo-1*H*-isoindol-4-yl]acetamide methylethyl ketone solvate crystals are collected or separated from their mother liquor by any conventional method such as filtration, centrifugation, evaporation of the solvent, decantation of the solvent or by two or more of these methods.

Finally, the isolated *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide methylethyl ketone solvate crystals may be dried at a temperature of about 100 °C or less, preferably of about 80 °C or less, more preferably of about 60 °C or less and most preferably the crystals are dried at a temperature of about 40 °C or less for example at about room temperature. Drying may be performed at ambient pressure and/or by applying vacuum. Drying is carried out at an atmosphere having a pressure of about 1013 mbar or less, preferably of about 100 mbar or less, more preferably of about 50 mbar or less and most preferably of about 30 mbar or less. Drying may be performed for a period of about 168 hours or less, preferably for about 84 hours or less, more preferably for about 48 hours or less and most preferably for about 24 hours or less.

In another embodiment, the crystalline solvates of the present invention can be used in medicine. The crystalline solvates may be used alone or in combination, or formulated with one or more excipients or other active pharmaceutical ingredients to provide formulations suitable for the treatment or prevention of such as psoriatric arthritis, plaque psoriasis, ankylosing spondylitis, Behcet's disease, rheumatoid arthritis, chronic prostatitis, chronic pelvic pain syndrome, vulvodynia, Crohn's disease, ulcerative colitis.

The administration form can be suitably chosen, e.g. a form suitable for oral, parenteral, rectal administration and/or administration by inhalation, and the dosage form may be solid, liquid, or powdery. Therefore, the pharmaceutical composition comprising the crystalline solvates of the present invention may suitably be in the form of tablets, pills, capsules, syrups, powders or granules for oral administration; or as sterile parenteral or subcutaneous solutions, suspensions for parenteral administration; or as suppositories for rectal administration.

Suitable excipients and/or carriers include, without being limited to, diluents, binders, disintegrants, lubricants, etc. For example, the crystalline solvates of the present invention, or particles comprising said crystalline solvates, are mixed with a carrier or binder substance, e.g. a mono-, di- or polysaccharide such as sugars and starch, a sugar alcohol or another polyol. For example, lactose, saccharose, sorbitol, mannitol, starch, cellulose derivatives, a binder such as polyvinylpyrrolidone, and a lubricant such as magnesium stearate, calcium stearate, polyethylene glycol, waxes, paraffin, and the like are mixed, and then compressed into tablets. The crystalline solvates of the present invention or particles containing the same may be coated by another substance. The powder mixture or particles containing the crystalline solvates of the present invention may also be dispensed into capsules.

WO 2009/120167 A1 discloses several crystalline solvates of *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide such as a toluene solvate (form C), a methylene chloride solvate (form D), an acetonitrile solvate (form E), an ethyl acetate solvate (form G) and a tetrahydrofuran solvate (form H). The present invention provides crystalline solvates of *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide with different organic solvents for example an ethanol solvate (form S_{EtOH}), a n-propanol solvate (S_{n-PrOH}), an acetone solvate (S_{ACT}) and a methylethyl ketone solvate (S_{MEK}).

According to the ICH HARMONIZED TRIPARTITE GUIDELINE, impurities: guideline for residual solvents Q3C(R5), current step 4 version, February 4th, 2011 some solvents that are known to cause unacceptable toxicities (ICH class 1 solvents) should be avoided in the production of drug substances, excipients or drug products unless their use can be strongly justified in a risk-benefit assessment. Some solvents associated with less severe toxicity (ICH class 2 solvents) should be limited in order to protect patients from potential adverse effects. The guideline further teaches that less toxic solvents (ICH class 3 solvents) should be used where practical.

In contrast to the solvates described in WO 2009/120167 A1, where the majority of the solvates contains a class 2 solvent, the solvates of the present invention without exception contain less toxic ICH class 3 solvents and are therefore preferred to be used in pharmaceutical products.

The only solvate described in WO 2009/120167 A1 containing an ICH class 3 solvent is the ethyl acetate solvate (form G). Organic solvents are expensive and harmful to the environment, that's why they should be reduced to the extent possible, preferably by partly replacing them with water. Hence, the use of water-miscible organic solvents is preferred for the final crystallization step. However, the water solubility of ethyl acetate is very limited (aqueous solubility 8.7% w/w, see table 1 herein), which is a disadvantage with regards to ecology and economy of the industrial production process. In contrast, the solvates of the present invention are crystallized from organic solvents with a water solubility of more than 20 % w/w, respectively solvents like ethanol, n-propanol and acetone are freely soluble in water. Hence, the amount of organic solvents applied for the production of the solvates of the present invention can be significantly reduced by simply increasing the water content of the solvent system. This renders industrial production processes of the novel solvates of the present invention cost-efficient and environmentally friendly.

**Table 1: ICH classification and water solubility of organic solvents incorporated into the crystal lattice of N-[2-[(1S)-1-(3-ethoxy-4-methoxyphenyl)-2-(methylsulfonyl)ethyl]-2,3-dihydro-1,3-dioxo-1H-isoindol-4-yl]acetamide**

| **Form** | **First mentioned in** | **Solvent incorporated into crystal structure** | **ICH classification** | **solubility in H₂O [%w**/**w]** |
|---|---|---|---|---|
| C | WO 2009/120167 A1 | toluene | 2 | 0.051 |
| D | WO 2009/120167 A1 | methylene chloride | 2 | 1.6 |
| E | WO 2009/120167 A1 | acetonitrile | 2 | 100 |
| G | WO 2009/120167 A1 | ethyl acetate | 3 | 8.7 |
| H | WO 2009/120167 A1 | tetrahydrofuran | 2 | 100 |
| S_{EtOH} | present invention | ethanol | 3 | 100 |
| S_{n-PrOH} | present invention | n-propanol | 3 | 100 |
| S_{ACT} | present invention | acetone | 3 | 100 |
| S_{MEK} | present invention | methylethyl ketone | 3 | 24 |

In addition, WO 2009/120167 A1 provides information about the morphology and the particle size respectively particle size distribution of the solvates. This information is once again summarized in table 2 of the present invention.

**Table 2: Summary of information provided in WO 2009/120167 A1 concerning particle size distribution and morphology**

| **Form** | **First mentioned in** | **Solvent incorporated into crystal structure** | **D₉₀** | **morpholgy** |
|---|---|---|---|---|
| C | WO 2009/120167 A1 | toluene | < 12 microm | plate-like |
| D | WO 2009/120167 A1 | methylene chloride | < 18 microm | flake-like |
| E | WO 2009/120167 A1 | acetonitrile | < 18 microm | flake-like |
| G | WO 2009/120167 A1 | ethyl acetate | < 18 microm | flake-like |
| H | WO 2009/120167 A1 | tetrahydrofuran | not disclosed | not disclosed |

As mentioned in WO 2009/120167 A1 (see page 26, lines 22-24) the D₉₀ value represents the 90^{th} percentile of the particle size distribution as measured by length or in other words, 90% of the particles have a length of this value or less. As can be seen from table 2 herein, the so far known crystalline solvates of *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide mainly consist of very small particles, such as particles having a particle size of less than about 20 microm. This is challenging for pharmaceutical development of a drug product as such small particles are usually highly static prone and therefore tend to agglomerate to larger particles and/ or to stick on surfaces during drug product manufacturing and have poor flow properties.

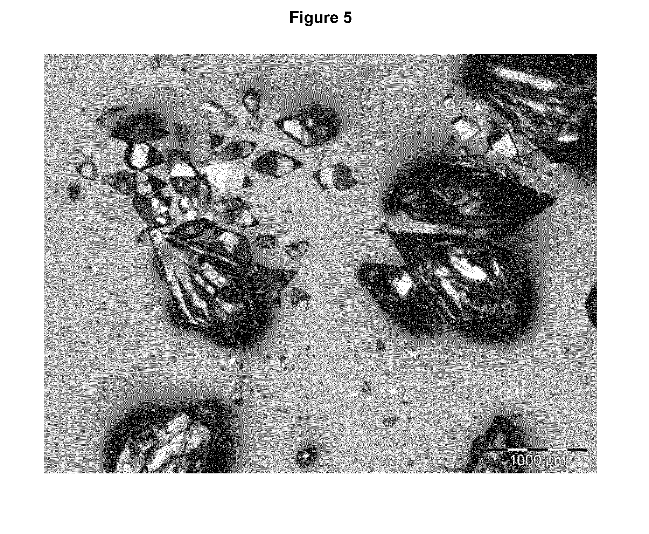

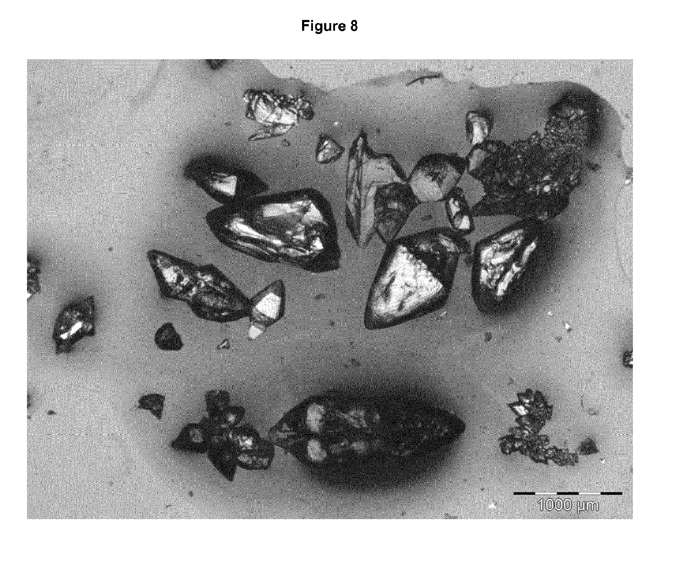

In contrast, the novel crystalline solvates of the present invention consist of significantly larger particles as can for example be seen from figures 5 and 8 for the ethanol solvate and the n-propanol solvate, respectively. The availability of solvates with increased particle size is associated with improved powder characteristics such as improved flow properties, less static charge and therefore fewer tendencies for agglomeration and/ or sticking on surfaces. In addition, larger crystals can be easier isolated from their mother liquor, for example filtration times can be decreased. Hence, the solvates of the present invention are associated with improved properties favoring them for pharmaceutical production processes.

The powder characteristics, in particular the flow properties also depend on the crystal shape. The crystalline solvates of the present invention show improved morphology compared to the solvates disclosed in WO 2009/120167 A1, which show either flake-like or plate-like morphology. For example, the ethanol solvate and the n-propanol solvate of the present invention crystallize as bipyramidal crystals, almost having spherical shape (see figures 6 and 8 herein). Powders, comprising such particles are characterized by good flow properties and are therefore favorable for pharmaceutical production processes.

The present invention is further illustrated by the following non-limiting examples.

### EXAMPLES

PXRDs were obtained with an X'Pert PRO diffractometer (PANalytical, Almelo, The Netherlands) equipped with a theta/theta coupled goniometer in transmission geometry, programmable XYZ stage with well plate holder, Cu-Kα_{1,2} radiation source (wavelength 0.15419 nm) with a focussing mirror, a 0.5° divergence slit, a 0.02° soller slit collimator and a 0.5° anti-scattering slit on the incident beam side, a 2 mm anti-scattering slit, a 0.02° soller slit collimator, a Ni-filter and a solid state PIXcel detector on the diffracted beam side. The diffractograms were recorded at room temperature at a tube voltage of 40 kV, tube current of 40 mA, applying a stepsize of 0.013° 2-Theta with 40 sec per step in the angular range of 2° to 40° 2-Theta. A typical precision of the 2-Theta values is in the range of ± 0.2° 2-Theta. Thus, a reflection that appears for example at 7.4° 2-Theta can appear between 7.2 and 7.6° 2-Theta on most X-ray diffractometers under standard conditions.

Fourier transform infrared (FTIR) spectra were recorded using a Spectrum GX FT-IR-System spectrometer (Perkin Elmer, Norwalk Ct., USA) with a Durasampler ATR. The samples were measured at room temperature in reflection mode (spectral range (4000-600) cm⁻¹, resolution 2 cm⁻¹, 24 scans). To record a spectrum a spatula tip of a sample was applied to the surface of the ZnSe in powder form. Then the sample was pressed onto the ZnSe and the spectrum was recorded. A typical precision of the wavenumber values is in the range of about ± 2 cm⁻¹. Thus, an infrared peak that appears for example at 1696 cm⁻¹ can appear between 1694 and 1698 cm⁻¹ on most infrared spectrometers under standard conditions.

¹H-NMR spectra were recorded at 500 MHz on a Bruker Avance 500 instrument. Deuterated dimethyl sulfoxide (99.9%D) with tetramethylsilane as internal standard was used as solvent.

Gravimetric moisture sorption desorption isotherm was acquired using a SPS-11 moisture sorption analyzer (MD Messtechnik, Ulm, D). The sample was weighed into an Aluminium sample holder. The measurement cycle was started at 0% RH, increased to 10% RH in 5% steps, further increased in 10% steps to 90% RH and subsequently increased to 95% RH, decreased again to 90% RH, decreased in 10% steps to 10% RH and finally further decreased in 5% steps to 0% RH. The water content of the samples was determined after the moisture sorption/ desorption experiments with a TGA 7 system (Perkin Elmer, Norwalk, Ct., USA) using the Pyris 2.0 software according to the above described method.

Intensity data for the crystal structure determination were recorded at 293 K, using Mo radiation (wavelength 0.71073 Å), on an Oxford Diffraction Gemini-R Ultra diffractometer operated by the CrysAlis software [Agilent Technologies (2012), Yarnton, England]. The structure was solved using the direct methods procedure in SHELXS97 and refined by full-matrix least squares on *F*² using SHELXL97. The positions of all non-hydrogen atoms were located in difference maps and their thermal parameters were refined anisotropically. Data collection and refinement: 50370 reflections collected; 4932 independent reflections; 335 parameters; absolute structure parameter-0.08(12); final indices [*F*² > 2σ(*F*²)] *R*1 = 0.058 and *wR2* = 0.143; *R* indices (all data) *R*1 = 0.0864 and w*R2* = 0.1572).

The microscopic images were obtained with a OLYMPUS SZX12 polarized light microscope using an OLYMPUS DP71 camera.

### Example 1: Preparation of N-[2-[(1S)-1-(3-ethoxy-4-methoxyphenyl)-2-(methylsulfonyl)-ethyl]-2,3-dihydro-1,3-dioxo-1H-isoindol-4-yl]acetamide ethanol solvate

A mixture of 20 mg *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide (e.g. prepared according to example 2 of WO 2003/080049 A1) in 8 mL aqueous ethanol (50% v/v) was heated to reflux temperature, whereupon a solution was obtained. The hot solution was filtered, cooled to a temperature of about 0 to 5 °C by placing the vial containing the solution into an ice-water bath and subsequently allowed to stand at a temperature of about 0 to 10 °C in the fridge. Within a period of 5 to 10 days the ethanol solvate of *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide crystallized as bi-pyramidal crystals. The obtained crystals were collected by filtration and air dried overnight.

### Example 2: Preparation of N-[2-[(1S)-1-(3-ethoxy-4-methoxyphenyl)-2-(methylsulfonyl)-ethyl]-2,3-dihydro-1,3-dioxo-1H-isoindol-4-yl]acetamide n-propanol solvate

A mixture of 20 mg *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide (e.g. prepared according to example 2 of WO 2003/080049 A1) in 8 mL aqueous n-propanol (50% v/v) was heated to reflux temperature, whereupon a solution was obtained. The hot solution was filtered, cooled to a temperature of about 0 to 5 °C by placing the vial containing the solution into an ice-water bath and subsequently allowed to stand at a temperature of about 0 to 10 °C in the fridge. Within a period of 5 to 7 days the n-propanol solvate of *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide crystallized as bi-pyramidal crystals. The obtained crystals were collected by filtration and air dried overnight.

### Example 3: Preparation of N-[2-[(1S)-1-(3-ethoxy-4-methoxyphenyl)-2-(methylsulfonyl)-ethyl]-2,3-dihydro-1,3-dioxo-1H-isoindol-4-yl]acetamide n-propanol solvate

A mixture of 30 mg *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide (e.g. prepared according to example 2 of WO 2003/080049 A1) in 8 mL n-propanol was heated to reflux temperature, whereupon a solution was obtained. The hot solution was filtered, cooled to a temperature of about 0 to 5 °C by placing the vial containing the solution into an ice-water bath and subsequently allowed to stand at a temperature of about 0 to 10 °C in the fridge. Within a period of 7 to 10 days the n-propanol solvate of *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide crystallized as bi-pyramidal crystals. The obtained crystals were collected by filtration and air dried overnight.

### Example 4: Preparation of N-[2-[(1S)-1-(3-ethoxy-4-methoxyphenyl)-2-(methylsulfonyl)-ethyl]-2,3-dihydro-1,3-dioxo-1H-isoindol-4-yl]acetamide acetone solvate

A mixture of 45 mg *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide (e.g. prepared according to example 2 of WO 2003/080049 A1) in 4 mL acetone was dissolved at room temperature. The solution was filtered, cooled to a temperature of about 0 to 5 °C by placing the vial containing the solution into an ice-water bath and subsequently allowed to stand unsealed at a temperature of about 0 to 10 °C in the fridge until the solvent evaporated and an oily material was obtained. After slightly scratching with a spatula *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide acetone solvate crystals were obtained, which were air dried overnight.

### Example 5: Preparation of N-[2-[(1S)-1-(3-ethoxy-4-methoxyphenyl)-2-(methylsulfonyl)-ethyl]-2,3-dihydro-1,3-dioxo-1H-isoindol-4-yl]acetamide methylethyl ketone solvate

A mixture of 44 mg *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide (e.g. prepared according to example 2 of WO 2003/080049 A1) in 4 mL methylethyl ketone was heated to reflux temperature, whereupon a solution was obtained. The hot solution was filtered, cooled to a temperature of about 0 to 5 °C by placing the vial containing the solution into an ice-water bath and subsequently allowed to stand unsealed at a temperature of about 0 to 10 °C in the fridge until the solvent evaporated and an oily material was obtained. After slightly scratching with a spatula *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide methylethyl ketone solvate crystals were obtained, which were air dried overnight.

## Claims

1. A crystalline solvate of *N*-[2-[(1*S*)-1-(3-ethoxy-4-methoxyphenyl)-2-(methylsulfonyl)ethyl]-2,3-dihydro-1,3-dioxo-1*H*-isoindol-4-yl]acetamide and an organic solvent, wherein the organic solvent is selected from an ICH class 3 solvent which has a water solubility of at least 20% w/w when measured at a temperature in the range of from 15 to 25 °C.

2. The crystalline solvate according to claim 1, wherein the organic solvent is selected from the group consisting of ethanol, n-propanol, acetone and methyl ethyl ketone.

3. The crystalline solvate according to claim 1, wherein the organic solvent is ethanol and which is **characterized by** having a powder X-ray diffractogram comprising reflections at 2-Theta angles of (7.4 ± 0.2)°, (11.2 ± 0.2)°, (15.2 ± 0.2)°, (16.3 ± 0.2)°, (26.3 ± 0.2)°, when measured at a temperature in the range of from 15 to 25 °C with Cu-Kalpha_{1,2} radiation having a wavelength of 0.15419 nm.

4. The crystalline solvate according to claim 3, having a Fourier transform infrared spectrum comprising peaks at wavenumbers of (1762 ± 2) cm⁻¹, (1696 ± 2) cm⁻¹, (1618 ± 2) cm⁻¹, (1519 ± 2) cm⁻¹, (1135 ± 2) cm⁻¹, when measured at a temperature in the range of from 15 to 25 °C with a ZnSe ATR cell.

5. The crystalline solvate according to claim 1, wherein the organic solvent is n-propanol and which is **characterized by** having a powder X-ray diffractogram comprising reflections at 2-Theta angles of (7.4 ± 0.2)°, (11.2 ± 0.2)°, (15.2 ± 0.2)°, (16.3 ± 0.2)°, (26.3 ± 0.2)°, when measured at a temperature in the range of from 15 to 25 °C with Cu-Kalpha_{1,2} radiation having a wavelength of 0.15419 nm.

6. The crystalline solvate according to claim 5, having a Fourier transform infrared spectrum comprising peaks at wavenumbers of (1762 ± 2) cm⁻¹, (1696 ± 2) cm⁻¹, (1618 ± 2) cm⁻¹, (1520 ± 2) cm⁻¹, (1135 ± 2) cm⁻¹, when measured at a temperature in the range of from 15 to 25 °C with a ZnSe ATR cell.

7. The crystalline solvate according to claim 1, wherein the organic solvent is acetone and which is **characterized by** having a powder X-ray diffractogram comprising reflections at 2-Theta angles of (7.4 ± 0.2)°, (11.2 ± 0.2)°, (15.2 ± 0.2)°, (16.4 ± 0.2)° and (26.3 ± 0.2)°, when measured at a temperature in the range of from 15 to 25 °C with Cu-Kα_{1,2} radiation having a wavelength of 0.15419 nm.

8. The crystalline solvate according to claim 7, having a Fourier transform infrared spectrum comprising peaks at wavenumbers of (1762 ± 2) cm⁻¹, (1700 ± 2) cm⁻¹, (1618 ± 2) cm⁻¹, (1519 ± 2) cm⁻¹ and (1136 ± 2) cm⁻¹, when measured at a temperature in the range of from 15 to 25 °C with a ZnSe ATR cell.

9. The crystalline solvate according to claim 1, wherein the organic solvent is methylethyl ketone and which is **characterized by** having a powder X-ray diffractogram comprising reflections at 2-Theta angles of (7.4 ± 0.2)°, (11.2 ± 0.2)°, (15.1 ± 0.2)°, (16.3 ± 0.2)° and (26.2 ± 0.2)°, when measured at a temperature in the range of from 15 to 25 °C with Cu-Kα_{1,2} radiation having a wavelength of 0.15419 nm.

10. The crystalline solvate according to claim 9, having a Fourier transform infrared spectrum comprising peaks at wavenumbers of (1762 ± 2) cm⁻¹, (1695 ± 2) cm⁻¹, (1618 ± 2) cm⁻¹, (1519 ± 2) cm⁻¹ and (1136 ± 2) cm⁻¹, when measured at a temperature in the range of from 15 to 25 °C with a ZnSe ATR cell.

11. The crystalline solvate according to any one of claims 1 to 10, or mixtures thereof, for use as a medicament.

12. The crystalline solvate according to any one of claims 1 to 10, or mixtures thereof, for use in the treatment or prevention of a disease selected from the group consisting of psoriatric arthritis, plaque psoriasis, ankylosing spondylitis, Behcet's disease, rheumatoid arthritis, chronic prostatitis, chronic pelvic pain syndrome, vulvodynia, Crohn's disease, ulcerative colitis.

13. A pharmaceutical composition comprising the crystalline solvate according to any one of claims 1 to 10, or mixtures thereof, and at least one pharmaceutically acceptable excipient.
